(19) Europäisches Patentamt

European Patent Office

Office européen des brevets

(11) **EP 0 542 845 B1**

(12) **FASCICULE DE BREVET EUROPEEN**

(45) Date de publication et mention
de la délivrance du brevet:
**01.05.1996  Bulletin 1996/18**

(21) Numéro de dépôt: **91914543.3**

(22) Date de dépôt: **01.08.1991**

(51) Int Cl.$^6$: **C07K 14/00**, C12N 15/70,
A61K 39/015, G01N 33/569,
C07K 7/08

(86) Numéro de dépôt international:
**PCT/FR91/00639**

(87) Numéro de publication internationale:
**WO 92/02549 (20.02.1992 Gazette 1992/05)**

(54) **ANTIGENE DE PLASMODIUM FALCIPARUM CAPABLE D'INDUIRE DES ANTICORPS
PROTECTEURS, APPLICATION A LA VACCINATION**

SCHÜTZENDE ANTIKÖRPER ERREGENDE PLASMODIUM FALCIPARUM ANTIGENE UND
DEREN VERWENDUNG IN IMPFSTOFFEN

PLASMODIUM FALCIPARUM ANTIGEN FOR ELICITING PROTECTIVE ANTIBODIES FOR USE IN
VACCINES

(84) Etats contractants désignés:
**AT BE CH DE DK ES FR GB GR IT LI LU NL SE**

(30) Priorité: **06.08.1990 FR 9010039**

(43) Date de publication de la demande:
**26.05.1993  Bulletin 1993/21**

(73) Titulaire: **INSTITUT PASTEUR
F-75724 Paris Cédex 15 (FR)**

(72) Inventeurs:
• **PUIJALON, Odile
F-92130 Issy (FR)**
• **BONNEFOY, Serge
F-92110 Clichy (FR)**
• **BOUHAROUN, Hasnaa
F-75015 Paris (FR)**

• **DRUILHE, Pierre
F-75016 Paris (FR)**

(74) Mandataire: **Desaix, Anne et al
Ernest Gutmann - Yves Plasseraud S.A.
3, rue Chauveau-Lagarde
F-75008 Paris (FR)**

(56) Documents cités:
**EP-A- 0 112 784          WO-A-88/05785**

• **NUCLEIC ACIDS RESEARCH, vol. 13, no. 11,
1985, Arlington, VA (US); G. LANGSLEY et al.,
pp. 4191-4202**
• **INFECTION & IMMUNITY, vol. 55, no 6, juin 1987;
H. JOUIN et al., pp. 1387-1391**

## Description

L'invention a pour objet de nouveaux antigènes de <u>Plasmodium falciparum</u> et leur application notamment en vue de la protection contre le paludisme.

Les parasites responsables du paludisme chez l'homme, dont notamment <u>Plasmodium falciparum</u> ou <u>Plasmodium vivax</u> pour ne citer que les principaux d'entre eux, présentent chez l'hôte humain des morphologies différentes et expriment des antigènes différents en fonction de leur localisation dans l'organisme de l'hôte infecté. Les différences morphologiques et antigéniques de ces parasites au cours de leur cycle de vie chez l'homme, permettent de définir au moins quatre stades de développement distincts.

Le tout premier stade de développement du parasite chez l'homme correspond à la forme sporozoïte introduite dans le sang de l'hôte, par piqures d'insectes porteurs du parasite. Le second stade correspond au passage du parasite dans le foie et à l'infection des cellules hépatiques dans lesquelles les parasites se développent pour former les schizontes hépatiques qui lorsqu'ils sont matures (par exemple pour <u>P falciparum</u> le 6è jour après pénétration des sporozoïtes) libèrent par éclatement des mérozoïtes hépatiques. Le troisième stade est caractérisé par l'infection des érythrocytes sanguins par les formes asexuées (mérozoïtes) du parasite; ce stade érythrocytaire de développement correspond à la phase pathogène de la maladie. Le quatrième stade correspond à la formation des formes à potentiel sexué (ou gamétocytes) qui deviendront des formes sexuées ou gamètes extra-cellulaires chez le moustique.

On sait que de nombreuses études ont été entreprises pour isoler à partir des souches de parasites infectantes pour un hôte humain des fractions polypeptidiques, d'une part pour assurer le diagnostic <u>in vitro</u> du paludisme par détection des anticorps correspondants, et d'autre part, pour tenter de vacciner contre le paludisme.

Plusieurs études ont été réalisées sur les antigènes de <u>Plasmodium falciparum</u> et en particulier sur différents antigènes ayant un poids moléculaire (PM) proche de 100kDa (fraction 100kDa). Des résultats ont montré que la fraction 100kDa est constituée de plusieurs antigènes distincts, migrant dans la même zone de poids moléculaire mais formant des taches (spots) différentes après séparation selon le point isoélectrique. Des résultats obtenus en 1982 ont montré que les singes Saimiri immuns et protégés après infection par <u>P. falciparum</u> ont des anticorps reconnaissant des antigènes migrant en gel de polyacrylamide-SDS avec un PM apparent de 96-100kDa. La présence de cette spécificité est liée à l'activité protectrice du sérum. Les singes immunisés par infection mais non protégés ne possèdent pas de tels anticorps (Gysin et al 1982 Parasite immunol 4/ 421).

On a aussi constaté que la fraction d'antigènes parasitaires obtenue par élution de la bande de P.M. d'environ 100kDa de gels de polyacrylamide-SDS préparatifs conférait par immunisation à des singes Saimiri une protection appréciable contre une infection à <u>P. falciparum</u> (Dubois et al 1984 Proc Natl Acad Sci 81229).

Trois taches distinctes (pl 5,15; 5,25 et 5,35) ont été mises en évidence par Jouin et al (1987, Infec Immun, 55, 1387); l'une d'elles correspond à l'antigène 96 TR, antigène thermostable reconnu préférentiellement par des sérums d'adultes immuns mais non par des enfants vivant en zone d'endémie.

Tous les antigènes décrits jusqu'à présent dans la fraction 100kDa sont différents par leur séquence primaire.

C'est le cas de l'antigène GBP 130 de Ravetch (Ravetch et al 1985, Science 227, 1593; Kochan et al 1986 Cell 44, 689) qui correspond à l'antigène 96 TR cité plus haut, de l'antigène 44 (Cooper et al 1988, Molec Biochem Parasitol 29, 251) isolé à partir des rhoptries et reconnu par les anticorps d'individus exposés à <u>P. falciparum</u> en zone d'endémie palustre. Un autre antigène de cette catégorie est ABRA/Ag 101 (Weber et al, 1988, J Biol Chem 263, 11421).

La présente invention concerne un antigène de <u>P falciparum</u>, présentant des réactions immunologiques croisées avec des antigènes appartenant à la fraction 100kDa mais qui se distingue des précédents notamment par sa séquence primaire. L'antigène selon l'invention est en outre susceptible d'induire des anticorps et en particulier des anticorps protecteurs vis à vis de <u>P falciparum</u>.

L'invention vise par ailleurs l'utilisation de ces antigènes dans des compositions vaccinantes contre <u>P falciparum</u> ainsi que pour la détection <u>in vitro</u> d'une infection due à <u>P falciparum</u>.

Font également partie de l'invention les séquences nucléotidiques codant pour l'antigène ci-dessus, en particulier les séquences d'ADN simple et double brins ainsi que les ARN correspondant.

Les antigènes de l'invention peuvent être isolés et purifiés à partir de <u>P falciparum</u> ou peuvent être produits par synthèse chimique en utilisant les méthodes classiques de production.

L'invention concerne donc une séquence nucléotidique de <u>P falciparum</u>, caractérisée en ce qu'elle comprend un enchaînement de nucléotides comportant n répétitions successives de fragments nucléotidiques identiques ou non de 18 paires de bases (bp) codant pour des peptides, identiques ou non, de 6 acides aminés répondant au motif de base suivant Asp b Asn His Lys Ser dans lequel b est un acide aminé quelconque, ou codant pour une variante de ce motif de base obtenue par substitution d'un ou plusieurs acides aminés dès lors que les propriétés antigéniques et/ou immunogènes de l'oligopeptide formé ne sont pas modifiées, n étant suffisant pour que l'enchaînement nucléotidique formé code pour un oligopeptide capable d'induire <u>in vivo</u> des anticorps qui reconnaissent l'antigène codé par la séquence nucléotidique de <u>P falciparum</u> répondant à la carte de restriction donnée ci-après.

GENE R45

A  : Alu I
Ac : Acc I
R  : Rsa I
✱  : codon stop
Rp :  ≈ 7 répétitions 45 bp

De préférence, la séquence nucléotidique ci-dessus code pour un oligopeptide capable d'induire _in vivo_ des anticorps susceptibles d'être protecteurs ou impliqués dans le mécanisme de protection contre P falciparum chez l'homme.

De tels anticorps dits "anticorps protecteurs" sont efficaces dans un test d'inhibition cellulaire dépendante d'anticorps (ADCI). Dans ce test la cellule effectrice (monocyte de sujet sain), en présence d'anticorps spécifiques, inhibe la culture de P falciparum. Une étroite corrélation a été établie entre l'effet inhibiteur des anticorps en ADCI et leur pouvoir protecteur _in vivo_ chez les sujets infectés.

Le test de l'ADCI peut par exemple être effectué dans les conditions suivantes :

des cellules mononuclées du sang de donneurs français sains ont été séparées sur un gradient de densité Ficoll-Hypaque® (Pharmacia) selon la technique de BÖYUM (1968 Scand J Clean Lab. Invest. 21 Suppl. 97: 77). Le nombre de monocytes dans la suspension cellulaire obtenue a été estimé par une coloration de l'estérase non spécifique (NSE) (TUCKER et al J immunol Meth 14: 267). La suspension cellulaire mononuclée (dans un milieu RPMI) a été ensuite distribuée dans des plaques de 96 trous suivant un taux de $2.10^5$ monocytes (cellules $NSE^+$) par puits. Après 90 Minutes d'incubation à 37°C dans de l'air contenant 5% de $CO_2$ les cellules non adhésives ont été retirées des puits en lavant avec du RPMI. Cette méthode a permis de récupérer $10^5$ cellules adhésives par puits parmi lesquelles plus de 90% étaient des monocytes.

Dans les puits contenant les monocytes adhésifs, des cultures asynchrones de P falciparum ont été ajoutées suivant un taux de 200 RBC/I monocyte. Le milieu de culture (RPMI + 10% de sérum humain) a été complété avec chacune des IgG purifiées à tester à 10% de leur concentration initiale dans le sérum du donneur (ou 5,10,20 et 40% pour certaines expériences). Les puits de contrôle consistent en 1) une culture seule, 2) une culture et des IgG test sans monocyte, 3) une culture et des IgG de contrôle, 4) une culture et des IgG de contrôle et des monocytes. Le test a en principe duré 48 heures. La parasitémie commençant à environ 0,2% à 0,5% a généralement atteint 1% à 3%.

L'index d'inhibition de la croissance spécifique (SGI) qui prend en compte l'inhibition possible induite soit par les cellules soit par les anticorps (IgG) seuls, utilisé comme contrôle dans chaque expérience a été calculé comme suit :
SGI = 1 - [(pourcentage de parasites calculés avec monocytes et IgG divisé par le pourcentage de parasitémie avec IgG) divisé par le
(pourcentage de parasitémie avec monocytes et IgG contrôle/pourcentage de parasites avec que IgG contrôle)]
X 100.

L'invention vise aussi le gène de P falciparum contenant la séquence répondant à la définition ci-dessus et en particulier la séquence du fragment Ddel ci-dessous, codant pour un antigène présentant des réactions immunologiques croisées avec un antigène ayant un poids moléculaire d'environ 100kDa. Ce gène peut être isolé à partir de P falciparum, par exemple par mise en contact avec la séquence ci-dessus pour permettre une réaction d'hybridation dans des conditions suffisamment stringentes décrites plus loin.

Les acides aminés variables du motif de base décrit ci-dessus,sont choisis parmi les suivants, représentés par leur code :

Ala : alanine, A
Cys : cystéine, C
Asp : acide aspartique, D
Glu : acide glutamique, E
Phe : phénylalanine, F
Gly : glycine , G
His : histidine, H
Ile : isoleucine, I
Lys : lysine, K
Leu : leucine, L
Met : méthionine , M
Asn : asparagine, N
Pro : proline, P
Gln : glutamine , Q
Arg : arginine, R
Ser : sérine, S
Thr : thréonine, T
Val : valine, V
Trp : trytophane, W
Tyr : tyrosine, Y

Dans le cadre de la définition précédente, des séquences nucléotidiques préférées sont caractérisées en ce que les variantes du motif de base sont des séquences codant pour des peptides choisis indépendemment les uns des autres parmi les suivants :

```
Asp Ser Asn His Met Ser
Asp Ser Asn His Met Ser
    His Asn His Met Ser
Glu Ser Asn His Lys Ser
Asp Ser Asn His Glu Ser
```

D'autres variantes sont naturellement admises dès lors que l'oligopeptide résultant conserve les propriétés structurales et/ou fonctionnelles de l'oligopeptide codé par la séquence ci-dessus.

De préférence b est choisi parmi Ser, His, Asn, Cys pour former l'enchaînement Asp b Asn His Lys Ser.

Les antigènes de l'invention peuvent être isolés et purifiés à partir de P falciparum ou peuvent être produits par synthèse chimique en utilisant les méthodes classiques de production.

Une séquence nucléotidique préférée selon l'invention et répondant aux caractéristiques précédentes est la suivante :

DdeI
CT

GAG GAA TGT CAA GGG GAA GTT TAT TTA TTT GTT AAA AAG ATA CCT ATA

GAG GTA TGG ATA AGA CAG TAC AAC TTG ATG AAT GAT AAT GAT GGA GAA

TAT TTA TTA GAT GGG GAA AAT TTT ATT ATG GAA GCA GTA GCC TGC GCT

TAT TTA AGC GAG CAT TAT CCT GGA CTC ACA CCT AAA TTG TAC AAG GTA

TTA TAT GAG CCT GAA TGT GCT AAC TGT AAT GAA GAG GAT AAG AAT ATG

AGT GAA GAT AAT CAT AAG AAG GAT AGT AAA CAT AAG GGG GAT AGT AAT

CAC AAA AGT GAT AGT AAT CAC AAA AGT GAT AGT AAT CAC AAA AGT GAT

AGT AAT CAT AAA AGT GAT AGT AAT CAT AAA AGT GGT AGT AAT CAC AAA

AGT GAT TGT AAT CAC AAG AGT GGT AGT AAT CAC AAG AGT GGT AGT AAT

CAC AAG AGT GAT AGT AAT CAT CAA AGT GAT TGT AAT ... ... ... ...

**80 REPETITIONS 18bp**

... ... ... ... ... ... ... ... ... GAT CAT AAT CAC AAA AGC GAT

CAT AAC CAC AAG AGC GAT AAT AAC CAC AAG AGT GAT CAT AAT CAC AAA

AGT GAT CAT AAA AAA AAT AAT AAC AAT AAT AAG GAT AAT AAA AAT GAC

GAT AAT GAT GAC AGT GAT GCA AGC GAT GCA GTT GAT GAA GAT ATT GAG

ATA CTT GAG TCT TAT AGT GAT TTG AAT AAA TTT AAT GAG ATG TTA ACA

GAA CAA TTA AAT AAA AAT AAG GAT GGA TAT GTT GTT TTG GTT ACT GAA

TTA TTT GGT GAA GAT CTT TTT CAA TAT ATT AAT AAA AGG AAT GAA AAT

GAA GAT ACG CGT GTA AGA GAT GAA GAT AAA AAA ATA ATT ATG TTT GAA

TTT TTA AAG TTA TTA ATA AAA TTA CAT AAT GCA GGA TTG GTA CAT CTT

GAT ATA TCT CCT GAA AAT ATA TTG ATA GAA AAT AAT GGT GAG TTA CGC

TTA TGT GAT CTA GCT AAA TGT GCT CCA ATG TAT ACA CAC AAT TTA AGA

5

```
CAT ATT AAA GGA AAT GGA AAC GAT TTG TAT TCA TTT CAA TCT TGT CAA

CCT TGT GTT GGA AAA ATC CCA TGT ATT CCA AAA GAG TGT TGG GAT ATT

ATT AGA GAA CAT ATA AAA TTA AAA ATA GAT AAT CCT TTT GAA CAT TTA

TCA ACT ATT ACT GAT CAA GAA GAA AGA AAA AAA TAT TAT TTT GAT GTA

CAT TGT GTA GAT AAA TAT ATG CTT GGT ATA TTT TAT ATA TGG ATA TGG


AAT TTA AAT TAT ATA TGG AAA CGA GCT GAC CCA CCA AAT GAT AGA ACA

TTT AAT AAT TTC TTA AAA TAT AAT TTA AAT ATA AAT GTA TTT CAG TTA

GCC CAA CAA TGG CCA AAA GGT CTC AAA GAT ATA ATT AAC GTA AGA TAT

ATA AGA AAA AAA AAA AAA AAA AAA AAA ATA ATA ATA ATA ATA ATG ATG
                                                •
TTT TAT ATA GTA TGT GCA TAG TGAATGTTTT TTTTTATAT TTTAATACAT
                            * * *
•
GAAATGATAT ATATATATAT ATATATATAT ATATTTCTC TTTATAGAAA
TTATTAAGCC TAGAAAGTAG AATGAAGACA GACTTAAATG AATTAACTGA
ACATCCATGG TGGATTAATG AAGATTAATT TTAATTTTTT AAGTATTATA
TGAATATTTA TTATTAGTAG ACTTTATATA TAACAATAAA TATTGACACG
TGCAATATTA AAAAAAAAAA AAAAAAAAAA AAAAAAAGA ATTAAAAAGG
AATGTTTTAT TGTTTAGGAC TATATGGAAA AATAATAATA TATATATGTT
TCCACATTAA AAA
```

## * CODON STOP (3 PHASES)

Dans la séquence ci-dessus définie, les répétitions remplissent les conditions énoncées dans les pages précédentes.

Entre également dans le cadre de l'invention, toute partie ou fragment de la séquence ci-dessus codant pour un peptide, un oligopeptide ou une protéine ayant des propriétés antigéniques, voire immunogènes, seuls ou en association avec une molécule porteuse dans une composition ou sous forme d'une molécule hybride, lesdits fragments ayant de préférence la capacité d'induire la production d'anticorps en particulier protecteurs contre P falciparum. Ces fragments sont obtenus par exemple par coupure enzymatique à partir de la séquence totale ou par synthèse chimique.

A cet égard, l'invention porte notamment sur toute partie de la séquence nucléoditique codant pour un nombre de motifs de base répétés suffisant pour induire in vivo la formation d'anticorps de préférence d'anticorps contre P falciparum et de façon préférée cette partie est constituée par l'enchainement d'environ 80 répétitions répondant à la définition ci-dessus.

Selon un mode particulier de réalisation de l'invention, la séquence nucléoditique est caractérisée en ce qu'elle comprend l'enchainement R23 nucléoditique répondant à la formule suivante :

```
                                                              AC
AAA AGT GAT AGT AAC CAC AAG AGC GAT CAT AAT CAC AAG AGT GAT AGT

AAC CAC ATG AGC GAT CAT AAC CAC ATG AGC GAT CAT AAC CAC AAG AGC

GAT CAT AAC CAC AAG AGC GAT AAT AAC CAC AAA AGT GAT AGT AAC CAC

AAA AGT GAT AGT AAC CAC AAA AGT GAT AGT AAC CAC AAG AGC GAT CAT

AAT C
```

L'enchainement R23 qui appartient en tant que fragment individualisé à l'invention présente la propriété tout à fait avantageuse d'exprimer un polypeptide capable de fixer des anticorps cytophiles et de préférence d'induire des anticorps en particulier des anticorps protecteurs contre <u>P facliparum</u> lorsq'il est administré de façon à ce que les anticorps produits aient des propriétés cyctophiles. Des anticorps cytophiles sont aptes à se fixer par leur fragment Fc à des cellules en particulier des macrophages.

Selon un autre mode de réalisation de l'invention, la séquence nucléoditique est caractérisée en ce qu'elle comprend l'enchainement R45 répondant à la formule suivante :

```
R45         10        20        30        40        50        60
            ù         ù         ù         ù         ù         ù
        CACAAGAGCGATAATAACCACAAAAGTGATAGTAACCACAAAAGTGATAGTA


            70        80        90        100       110       12C
            ù         ù         ù         ù         ù         ù
    ACCACAAAAGTGATAGTAACCACAAGAGCGATCATAATCACAAGAGCGATCATAAGCACA


            130       140       150       160       170       180
            ù         ù         ù         ù         ù         ù
    TGAGCGATAATAACCACAAGAGCGATAATAATCACAAGAGCGATAATAATCACAAGAGCG


            190       200       210       220       230       240
            ù         ù         ù         ù         ù         ù
    ATCATAATCACAAGAGCGATAATAATCACAAGAGCGATCACAATCACAAGAGTGATAGTA


            250       260       270       280       290       300
            ù         ù         ù         ù         ù         ù
    ATCACAAGAGTGATAGTAACCACAAGAGTGATAGTAACCACAAGAGTGATAGTAACCACA


            310       320       330       340       350       360
            ù         ù         ù         ù         ù         ù
    AGAGTGATAATAACCATAAGAGCGATCATAATCAC[AACAGT]
```

L'invention vise aussi la séquence dépourvue du linker encadré sur le fragment R45 ci-dessus décrit (AACAGT) et qui correspond à la séquence suivante :

```
CAC AAG AGC GAT AAT AAC CAC AAA AGT GAT AGT AAC CAC AAA AGT GAT
1               15                  30                  45

AGT AAC CAC AAA AGT GAT AGT AAC CAC AAG AGC GAT CAT AAT CAC AAG
            60                  75                  90

AGC GAT CAT AAG CAC ATG AGC GAT AAT AAC CAC AAG AGC GAT AAT AAT
        105                 120                 135

CAC AAG AGC GAT AAT AAT CAC AAG AGC GAT CAT AAT CAC AAG AGC GAT
    150                 165                 180

AAT AAT CAC AAG AGC GAT CAC AAT CAC AAG AGT GAT AGT AAT CAC AAG
195                 210                 225                 240

AGT GAT AGT AAC CAC AAG AGT GAT AGT AAC CAC AAG AGT GAT AGT AAC
            255                 270                 285

CAC AAG AGT GAT AAT AAC CAT AAG AGC GAT CAT AAT CAC
        300                 315
```

Il est naturellement entendu que l'enchainement R45 en tant que tel est inclu dans cette définition.

Selon un autre mode de réalisation de l'invention, la séquence nucléoditique est caractérisée en ce qu'elle comprend l'enchaînement R51 répondant à la formule suivante :

```
        10      20      30      40      50      60
         ù       ù       ù       ù       ù       ù
R51
 [AATTC]ACAATCACATGAGCGATCATAATCACAAGAGTGATAATAACCATAAGAGTGATC


        70      80      90      100     110     120
         ù       ù       ù       ù       ù       ù
 ATAATCACAAGAGTGATAGTAACCACATGAGCGATCATAACCACATGAGCGATCATAACC


        130     140     150     160     170     180
         ù       ù       ù       ù       ù       ù
 ACAAGAGCGATCATAATCACAAGAGCGATCATAACCACAAGAGCGATAATAACCACAAAA


        190     200     210     220     230     240
         ù       ù       ù       ù       ù       ù
 GTGATAGTAACCACAAAAGT[GAGGAATTCC]
```

On remarquera que les séquences R23 et R45 se chevauchent.

De même l'invention concerne aussi le fragment R51 dépourvu du linker encadré en position 5'(AATTC) et dépourvu du linker en position 3'(GGAATTCC), correspondant à la séquence suivante:

```
CAC AAT CAC ATG AGC GAT CAT AAT CAC AAG AGT GAT AAT AAC CAT AAG
1               15                    30                    45

AGT GAT CAT AAT CAC AAG AGT GAT AGT AAC CAC ATG AGC GAT CAT AAC
            60                    75                    90

CAC ATG AGC GAT CAT AAC CAC AAG AGC GAT CAT AAT CAC AAG AGC GAT
        105                    120                    135

CAT AAC CAC AAG AGC GAT AAT AAC CAC AAA AGT GAT AGT AAC CAC AAA
        150                    165                    180

AGT GA
195
```

L'invention vise aussi les séquences ci-dessus auxquelles on aurait ajouté à une extrémité ou aux 2 extrémités des "linkers" (oligonucléotides synthétiques) tels que GGAATTCC.

L'invention vise aussi toutes les combinaisons des enchainements R23, R45 et R51, notamment la séquence formée des enchainements correspondant à R23 et R45.

De façon tout à fait avantageuse, la séquence de l'invention est caractérisée en ce qu'elle code pour un polypeptide capable d'induire la production d'anticorps cytophiles, de préférence d'isotypes $IgG_1$ ou $IgG_3$.

Elle concerne aussi la séquence nucléoditique qui comprend l'enchainement I en amont des 80 répétitions, et l'enchaînement II en aval des 80 répétitions, correspondant respectivement aux séquences suivantes :

I :                                                                    Dde I
                                                                        CT

GAG GAA TGT CAA GGG GAA GTT TAT TTA TTT GTT AAA AAG ATA CCT ATA

GAG GTA TGG ATA AGA CAG TAC AAC TTG ATG AAT GAT AAT GAT GGA GAA

TAT TTA TTA GAT GGG GAA AAT TTT ATT ATG GAA GCA GTA GCC TGC GCT

TAT TTA AGC GAG CAT TAT CCT GGA CTC ACA CCT AAA TTG TAC AAG GTA

TTA TAT GAG CCT GAA TGT GCT AAC TGT AAT GAA GAG GAT AAG AAT ATG

AGT GAA GAT AAT


II:                        AAA AAT AAT AAC AAT AAT AAG GAT AAT AAA AAT GAC

GAT AAT GAT GAC AGT GAT GCA AGC GAT GCA GTT GAT GAA GAT ATT GAG

ATA CTT GAG TCT TAT AGT GAT TTG AAT AAA TTT AAT GAG ATG TTA ACA

GAA CAA TTA AAT AAA AAT AAG GAT GGA TAT GTT GTT TTG GTT ACT GAA

TTA TTT GGT GAA GAT CTT TTT CAA TAT ATT AAT AAA AGG AAT GAA AAT

GAA GAT ACG CGT GTA AGA GAT GAA GAT AAA AAA ATA ATT ATG TTT GAA

TTT TTA AAG TTA TTA ATA AAA TTA CAT AAT GCA GGA TTG GTA CAT CTT

GAT ATA TCT CCT GAA AAT ATA TTG ATA GAA AAT AAT GGT GAG TTA CGC

TTA TGT GAT CTA GCT AAA TGT GCT CCA ATG TAT ACA CAC AAT TTA AGA

CAT ATT AAA GGA AAT GGA AAC GAT TTG TAT TCA TTT CAA TCT TGT CAA

CCT TGT GTT GGA AAA ATC CCA TGT ATT CCA AAA GAG TGT TGG GAT ATT

ATT AGA GAA CAT ATA AAA TTA AAA ATA GAT AAT CCT TTT GAA CAT TTA

TCA ACT ATT ACT GAT CAA GAA GAA AGA AAA AAA TAT TAT TTT GAT GTA

CAT TGT GTA GAT AAA TAT ATG CTT GGT ATA TTT TAT ATA TGG ATA TGG

```
AAT TTA AAT TAT ATA TGG AAA CGA GCT GAC CCA CCA AAT GAT AGA ACA

TTT AAT AAT TTC TTA AAA TAT AAT TTA AAT ATA AAT GTA TTT CAG TTA

GCC CAA CAA TGG CCA AAA GGT CTC AAA GAT ATA ATT AAC GTA AGA TAT

ATA AGA AAA AAA AAA AAA AAA AAA AAA ATA ATA ATA ATA ATA ATG ATG
                                        •
TTT TAT ATA GTA TGT GCA TAG TGAATGTTTT TTTTTTATAT TTTAATACAT
                            • • •
  •
GAAATGATAT ATATATATAT ATATATATAT ATATTTCTC TTTATAGAAA
TTATTAAGCC TAGAAAGTAG AATGAAGACA GACTTAAATG AATTAACTGA
ACATCCATGG TGGATTAATG AAGATTAATT TTAATTTTTT AAGTATTATA
TGAATATTTA TTATTAGTAG ACTTTATATA TAACAATAAA TATTGACACG
TGCAATATTA AAAAAAAAAA AAAAAAAAAA AAAAAAAGA ATTAAAAAGG
AATGTTTTAT TGTTTAGGAC TATATGGAAA AATAATAATA TATATATGTT
TCCACATTAA AAA
```

L'invention vise aussi toute partie de ces enchaînements, codant pour un antigène reconnu par des anticorps contre P falciparum ou susceptible d'induire ou de contribuer à la production in vivo de tels anticorps.

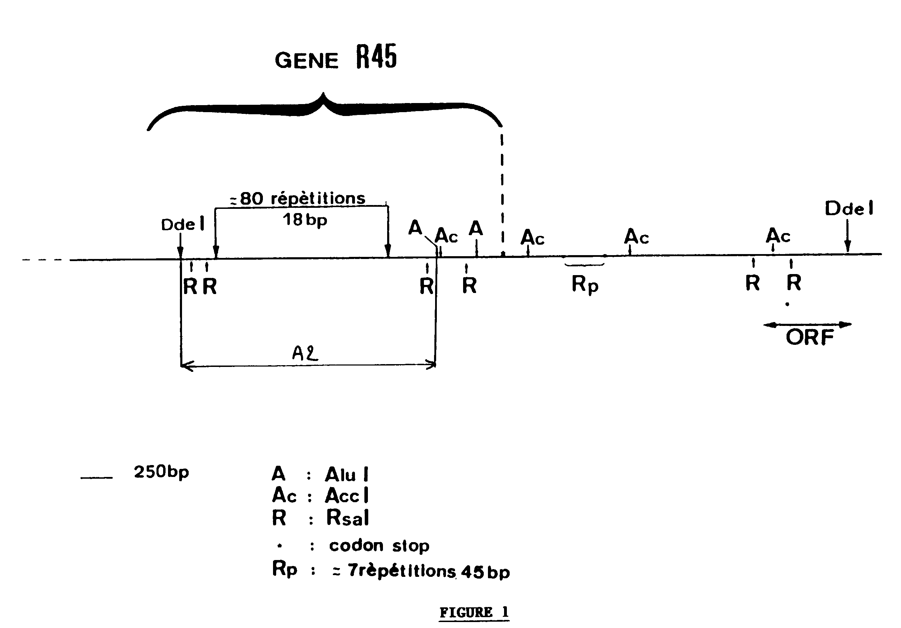

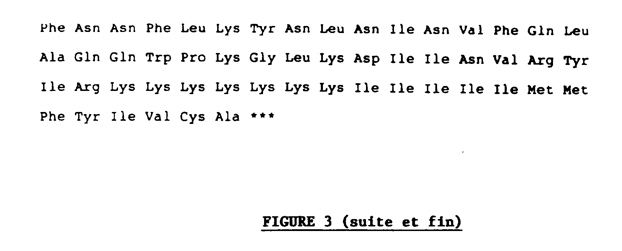

Un autre antigène selon l'invention est caractérisé en ce qu'il reconnu par des anticorps reconnaissant l'enchaînement polypeptidique codé par la séquence nucléotidique A2 située entre les sites de restriction Ddel en 5' et le premier site Alul en 3' après les 80 répétitions, de la séquence représentée sur la figure 1. Cet antigène a un poids moléculaire d'environ 160kDa et est présent à un stade précoce de l'infection par P falciparum. Cet antigène est aussi caractérisé en ce qu'il est reconnu par des anticorps dirigés contre la séquence de la figure 3. Cet antigène est décrit plus précisément à la partie VI des exemples et il peut être purifié par les techniques suivantes :

1) on suspend des bactéries contenant la protéine β-Gal recombinée avec la séquence d'acides aminés codée par A2, dans un tampon tel que le PBS, et on procède à une sonication dans des conditions permettant l'ouverture des bactéries recombinantes,

2) on centrifuge à 10 000 g, à 4°C pendant 10 minutes et on récupère le culot de centrifugation contenant la protéine A2-βgalactosidase recombinée,

3) on resuspend le culot de centrifugation avec un agent dénaturant tel que l'urée 6M,

4) on charge un gel préparatif de polyacrylamide-SDS, le gel étant à 7,5% de polyacrylamide et on laisse migrer quelques heures,

5) on procède à une coloration brève du gel au bleu de Coomassie, on décolore et on découpe la bande qui a migré à environ 200kDa et qui correspond à la protéine A2-βgal,

6) on électroélue cette bande de 200kDa au moyen d'un appareil de type BIORAD ELECTRO ELUTER modèle 422,

7) le cas échéant, on réanalyse en électrophorèse sur gel de polyacrylamide-SDS et on révèle le protéines par exemple par coloration au nitrate d'argent.

Entrent également dans le cadre de l'invention, les séquences nucléoditiques complémentaires des séquences précédentes ainsi que les séquences ayant subi des mutations ponctuelles, par exemple par substitution, délétion, insertion ou inversion de nucléotides, dès lors que les propriétés antigéniques, le cas échéant immunogènes des séquences correspondantes non modifiées sont conservées.

L'antigène de P falciparum conforme à l'invention, susceptible d'induire la formation d'anticorps, en particulier d'anticorps protecteurs contre P falciparum, est caractérisé en ce qu'il s'agit du produit de traduction de l'une des séquences traduites ci-dessus.

L'antigène de P falciparum ci-dessus est encore caractérisé en ce qu'il est susceptible d'induire des anticorps et en particulier des anticorps protecteurs contre P falciparum, et ce qu'il comprend une séquence d'acides aminés comportant un oligopeptide constitué de n répétitions de peptides identiques ou non répondant au motif de base Asp b Asn His Lys Ser dans laquelle b représente un acide aminé quelconque, ou à une variante de ce motif de base obtenue par substitution d'un ou plusieurs acides aminés dès lors que les propriétés antigéniques et/ou immunogènes de l'oligopeptide formé sont conservées, n étant tel que l'oligopeptide formé vérifie les propriétés énoncées ci-dessus par référence à la séquence nucléotidique.

Des variantes du motif de base peuvent être choisies parmi les peptides ci-dessus définis en rapport avec les séquences nucléotidiques de l'invention.

Dans un mode de réalisation particulier de l'invention, l'antigène répondant aux définitions ci-dessus répond à la séquence en acides aminés représentée ci-après :

Glu Glu Cys Gln Gly Glu Val Tyr Leu Phe Val Lys Lys Ile Pro Ile

Glu Val Trp Ile Arg Gln Tyr Asn Leu Met Asn Asp Asn Asp Gly Glu

Tyr Leu Leu Asp Gly Glu Asn Phe Ile Met Glu Ala Val Ala Cys Ala

Tyr Leu Ser Glu His Tyr Pro Gly Leu Thr Pro Lys Leu Tyr Lys Val

Leu Tyr Glu Pro Glu Cys Ala Asn Cys Asn Glu Glu Asp Lys Asn Met

Ser Glu Asp Asn His Lys Lys Asp Ser Lys His Lys Gly Asp Ser Asn

His Lys Ser Asp Ser Asn His Lys Ser Asp Ser Asn His Lys Ser Asp

Ser Asn His Lys Ser Asp Ser Asn His Lys Ser Gly Ser Asn His Lys

Ser Asp Cys Asn His Lys Ser Gly Ser Asn His Lys Ser Gly Ser Asn

His Lys Ser Asp Ser Asn His Gln Ser Asp Cys Asn ... ... ... ...

## 80 REPETITIONS : 6 aa

... ... ... ... ... ... ... ... ... Asp His Asn His Lys Ser Asp

His Asn His Lys Ser Asp Asn Asn His Lys Ser Asp His Asn His Lys

Ser Asp His Lys Lys Asn Asn Asn Asn Asn Lys Asp Asn Lys Asn Asp

Asp Asn Asp Asp Ser Asp Ala Ser Asp Ala Val Asp Glu Asp Ile Glu

Ile Leu Glu Ser Tyr Ser Asp Leu Asn Lys Phe Asn Glu Met Leu Thr

Glu Gln Leu Asn Lys Asn Lys Asp Gly Tyr Val Val Leu Val Thr Glu

Leu Phe Gly Glu Asp Leu Phe Gln Tyr Ile Asn Lys Arg Asn Glu Asn

Glu Asp Thr Arg Val Arg Asp Glu Asp Lys Lys Ile Ile Met Phe Glu

Phe Leu Lys Leu Leu Ile Lys Leu His Asn Ala Gly Leu Val His Leu

Asp Ile Ser Pro Glu Asn Ile Leu Ile Glu Asn Asn Gly Glu Leu Arg

Leu Cys Asp Leu Ala Lys Cys Ala Pro Met Tyr Thr His Asn Leu Arg

His Ile Lys Gly Asn Gly Asn Asp Leu Tyr Ser Phe Gln Ser Cys Gln

Pro Cys Val Gly Lys Ile Pro Cys Ile Pro Lys Glu Cys Trp Asp Ile

Ile Arg Glu His Ile Lys Leu Lys Ile Asp Asn Pro Phe Glu His Leu

Ser Thr Ile Thr Asp Gln Glu Glu Arg Lys Lys Tyr Tyr Phe Asp Val

His Cys Val Asp Lys Tyr Met Leu Gly Ile Phe Tyr Ile Trp Ile Trp

Asn Leu Asn Tyr Ile Trp Lys Arg Ala Asp Pro Pro Asn Asp Arg Thr

Phe Asn Asn Phe Leu Lys Tyr Asn Leu Asn Ile Asn Val Phe Gln Leu

Ala Gln Gln Trp Pro Lys Gly Leu Lys Asp Ile Ile Asn Val Arg Tyr

Ile Arg Lys Lys Lys Lys Lys Lys Lys Ile Ile Ile Ile Ile Met Met

Phe Tyr Ile Val Cys Ala ...

L'invention concerne également les parties de la séquence antigénique présentée précédemment; en particulier entrent dans le cadre de l'invention, les fragments antigéniques A et B situés en amont de l'enchainement comportant les 80 répétitions et en aval de cet enchainement.

**A :**

```
Glu Glu Cys Gln Gly Glu Val Tyr Leu Phe Val Lys Lys Ile Pro Ile
Glu Val Trp Ile Arg Gln Tyr Asn Leu Met Asn Asp Asn Asp Gly Glu
Tyr Leu Leu Asp Gly Glu Asn Phe Ile Met Glu Ala Val Ala Cys Ala
Tyr Leu Ser Glu His Tyr Pro Gly Leu Thr Pro Lys Leu Tyr Lys Val
Leu Tyr Glu Pro Glu Cys Ala Asn Cys Asn Glu Glu Asp Lys Asn Met
Ser Glu Asp Asn
```

**B :**

```
                Lys Asn Asn Asn Asn Asn Lys Asp Asn Lys Asn Asp
Asp Asn Asp Asp Ser Asp Ala Ser Asp Ala Val Asp Glu Asp Ile Glu
Ile Leu Glu Ser Tyr Ser Asp Leu Asn Lys Phe Asn Glu Met Leu Thr
Glu Gln Leu Asn Lys Asn Lys Asp Gly Tyr Val Val Leu Val Thr Glu
Leu Phe Gly Glu Asp Leu Phe Gln Tyr Ile Asn Lys Arg Asn Glu Asn
Glu Asp Thr Arg Val Arg Asp Glu Asp Lys Lys Ile Ile Met Phe Glu
Phe Leu Lys Leu Leu Ile Lys Leu His Asn Ala Gly Leu Val His Leu
Asp Ile Ser Pro Glu Asn Ile Leu Ile Glu Asn Asn Gly Glu Leu Arg
Leu Cys Asp Leu Ala Lys Cys Ala Pro Met Tyr Thr His Asn Leu Arg
His Ile Lys Gly Asn Gly Asn Asp Leu Tyr Ser Phe Gln Ser Cys Gln
Pro Cys Val Gly Lys Ile Pro Cys Ile Pro Lys Glu Cys Trp Asp Ile
Ile Arg Glu His Ile Lys Leu Lys Ile Asp Asn Pro Phe Glu His Leu
Ser Thr Ile Thr Asp Gln Glu Glu Arg Lys Lys Tyr Tyr Phe Asp Val
His Cys Val Asp Lys Tyr Met Leu Gly Ile Phe Tyr Ile Trp Ile Trp
Asn Leu Asn Tyr Ile Trp Lys Arg Ala Asp Pro Pro Asn Asp Arg Thr

Phe Asn Asn Phe Leu Lys Tyr Asn Leu Asn Ile Asn Val Phe Gln Leu
Ala Gln Gln Trp Pro Lys Gly Leu Lys Asp Ile Ile Asn Val Arg Tyr
Ile Arg Lys Lys Lys Lys Lys Lys Ile Ile Ile Ile Ile Met Met
Phe Tyr Ile Val Cys Ala ***
```

13

D'autres fragments également intéressants dans le cadre de l'invention sont les fragments constitués par tout ou partie des répétitions des peptides dont la définition a été donnée précédemment.

D'autres antigènes P falciparum s'inscrivant dans les définitions ci-dessus, sont les antigènes comprenant ou correspondant aux séquences d'acides aminés R23, R45 ou R51 et répondant respectivement aux enchainements d'acides aminés suivants :

**R23**

HisLysSerAspSerAsnHisLysSerAspHisAsnHisLysSerAspSerAsn

HisMetSerAspHisAsnHisMetSerAspHisAsnHisLysSerAspHisAsnHisLys

SerAspAsnAsnHisLysSerAspSerAsnHisLysSerAspSerAsnHisLysSerAsp

SerAsnHisLysSerAspHisAsn

**R51**

HisAsnHisMetSerAspHisAsnHisLysSerAspAsnAsnHisLysSerAspHis

AsnHisLysSerAspSerAsnHisMetSerAspHisAsnHisMetSerAspHisAsnHis

LysSerAspHisAsnHisLysSerAspHisAsnHisLysSerAspAsnAsnHisLysSer

AspSerAsnHisLysSer

**R45**

HisLysSerAspAsnAsnHisLysSerAspSerAsnHisLysSerAspSerAsn

HisLysSerAspSerAsnHisLysSerAspHisAsnHisLysSerAspHisLysHisMet

SerAspAsnAsnHisLysSerAspAsnAsnHisLysSerAspAsnAsnHisLysSerAsp

HisAsnHisLysSerAspAsnAsnHisLysSerAspHisAsnHisLysSerAspSerAsn

HisLysSerAspSerAsnHisLysSerAspSerAsnHisLysSerAspSerAsnHisLys

SerAspAsnAsnHisLysSerAspHisAsnHis

L'enchaînement R23 peut aussi être représenté par la séquence donnée ci-dessus, dans laquelle l'acide aminé initial en position 5' est la lysine qui suit l'histidine présentée comme premier acide aminé dans la séquence ci-dessus.

L'invention vise aussi des séquences peptidiques caractéristiques de P falciparum, reconnues par des anticorps dirigés contre P falciparum, et le cas échéant capable d'induire la formation d'anticorps contre P falciparum, ou de contribuer à la formation de tels anticorps, et en particulier la séquence suivante HKSDNNHKSDSNHKSDSN.

Selon un autre aspect de l'invention, les séquences nucléotidiques dont la description figure dans les pages précédentes, peuvent se présenter sous la forme d'un ADN recombinant. Dans ce cas la séquence nucléotidique caractéristique de P falciparum selon l'invention, est de préférence classée sous le contrôle d'un promoteur de préférence un promoteur fort, éventuellement inductible tel que celui de l'opéron lactose ou le promoteur pL ou pR du bactériophage lambda et d'éléments de régulation adaptés à son expression dans un hôte cellulaire choisi, tels que des signaux de terminaison, de transcription, d'excrétion ou de sécrétion.

L'invention concerne donc également un vecteur recombinant de clonage et/ou d'expression caractérisé en ce qu'il comprend une séquence nucléotidique telle que décrite précédemment, le cas échéant sous le contrôle d'un promoteur et des éléments de régulation pour l'expression dans un hôte cellulaire déterminé.

Des vecteurs particulièrement adaptés pour la réalisation de l'invention sont par exemple des plasmides, des cosmides ou des phages et en particulier le Baculovirus, λgt11, pMS notamment pMS23, pGEX.

L'invention vise aussi un polypeptide recombinant, caractérisé en ce qu'il comprend une séquence antigénique répondant aux définitions ci-dessus, en association avec un polypeptide hétérologue, notamment un polypeptide caractéristique d'un stade sanguin ou hépatique de l'infection par P falciparum, par exemple l'antigène LSA ou encore un polypeptide ou une protéine porteuse.

Par l'expression "polypeptide" il faut entendre tout peptide, polypeptide, protéine ou fragment protéique utilisable pour la réalisation de l'invention, en particulier tout polypeptide physiologiquement acceptable.

A titre d'exemple de protéine porteuse, on pourra citer la β-Galactosidase, l'anatoxine tétanique, l'ovalbumine, la sérum albumine, l'hémocyanine, le PPD de la tuberculine (Purified Protein Derivative), la glutathione transférase GT telle que celle de Schistosoma japonicum codée par le vecteur décrit par Smith D.B et Johnson K.S, (1988 Gene 67: 31). Des molécules porteuses synthétiques telles que les polylysines, les poly (D-L-alanine) - poly (L-lysine) sont également compatibles avec la réalisation de l'invention.

Un tel polypeptide recombinant, peut être utilisé pour la réalisation d'une composition vaccinante, par exemple lorsque la séquence antigénique de P falciparum qu'il contient ne permet pas en tant que telle de déclencher la production d'anticorps.

La séquence hétérologue peut être choisie de façon telle qu'elle stimule la formation des anticorps recherchés.

Par ailleurs la séquence polypeptidique hétérologue peut être choisie de façon telle qu'elle permet elle-même d'obtenir une réaction immunogène et qu'elle permet dans ce cas de déclencher la production d'anticorps. La séquence polypeptidique hétérologue peut par exemple être caractéristique d'un antigène de P falciparum par exemple d'un antigène de stade sanguin ou hépatique. On peut ainsi obtenir un effet conjugué et une réponse vis à vis d'antigènes différents.

Entre également dans le cadre de l'invention, un hôte cellulaire recombinant, caractérisé en ce qu'il est tranformé par un acide nucléique recombinant ou un vecteur recombinant tel que décrit dans les pages précédentes, dans des conditions permettant l'expression d'une séquence de P falciparum selon l'invention. Avantageusement le vecteur ou l'acide nucléique recombinant introduit est choisi pour sa compatibilité avec l'hôte cellulaire recombinant et est tel que la séquence de P falciparum est sous le contrôle d'un promoteur et le cas échéant des séquences codant pour les signaux de terminaison de la transcription, compatibles avec l'expression dans l'hôte choisi. A titre d'exemple d'hôte cellulaire utilisable pour réaliser l'invention, on peut citer les bactéries telles que E.coli, Bacillus subtilis ou des Streptomyces, les levures telles que saccharomyces cerevisiae, des champignons, les cellules d'insectes ou encore les cellules de mammifères telles que les cellules CHO.

Des hôtes cellulaires particulièrement intéressants pour la réalisation de l'invention sont d'une part la souche SB275 comprenant la séquence R23,déposée à la CNCM (Collection Nationale des cultures de microorganismes à Paris) sous le numéro I-986 le 27 Juillet 1990 et d'autre part la souche SB363 comprenant la séquence comprise entre les sites de restriction DdeI figurant sur la carte de restriction, déposée à la CNCM sous le numéro I-987 le 27 Juillet 1990.

Le bactériophage recombinant λgt11R45 est hébergé sous forme lysogène dans la souche SB95 déposée à la CNCM LE 27 Juillet 1990, sous le numéro I-985.

L'invention vise naturellement les séquences nucléotidiques de P falciparum contenues dans les vecteurs recombinants insérés dans les souches SB275 et SB363.

L'invention concerne aussi la souche SB95 contenant la séquence nucléotidique R45 décrite dans les pages précédentes, ainsi que cette séquence telle qu'issue du clone SB95.

L'invention permet aussi de réaliser une composition vaccinante, caractérisée en ce qu'elle comprend à titre de principe actif, un antigène répondant aux définitions ci-dessus.

Cette composition peut être obtenue par le mélange dudit principe actif, avec un véhicule pharmaceutique acceptable, choisi en fonction du mode d'administration souhaité.

Les résultats obtenus in vivo et in vitro par les inventeurs ont permis de déterminer qu'une utilisation de la composition vaccinante ci-dessus pour la production d'anticorps protecteurs contre P falciparum, doit tenir compte du mécanisme de coopération entre macrophages, monocytes et anticorps (processus de l'ADCI) (Antibody-Dependant-Cellular- Inhibitory effet) tel que décrit dans les exemples) mis en évidence au cours des expériences. Ainsi pour obtenir des anticorps protecteurs contre P falciparum, l'immunisation avec un antigène selon l'invention doit être réalisée de façon à induire préférentiellement des anticorps cytophiles (anticorps ayant une affinité pour diverses cellules en particulier de la lignée macrophagique) du type IgG$_1$ et IgG$_3$. Au contraire l'immunisation doit induire peu d'anticorps non cytophiles du type IgG$_2$, IgG$_4$ et IGM.

Les inventeurs ont donc mis en évidence que le mécanisme d'action protectrice est fonctionnel lorsque l'anticorps produit a une spécificité donnée, en particulier vis à vis des antigènes de l'invention, et lorsque cet anticorps appartient à un isotype susceptible de coopérer avec les monocytes.

Une autre composition vaccinante selon l'invention peut, outre le principe actif constitué par l'antigène de P falciparum défini précédemment, comporte un autre antigène caractéristique par exemple des stades sanguins ou hépatiques de P falciparum.

L'invention a aussi pour objet des amorces d'ADN ou d'ARN utilisables par exemple dans le cadre de la synthèse de séquences nucléotidiques éventuellement suivie de la synthèse polypeptidique selon l'invention par la technique de la PCR (Polymerase Chain Reaction) telle que décrite dans les brevets américains numéros 4.683.202 et 4.683.195 et dans la demande de brevet européen numéro 200.362 (PCR = amplification en chaine de l'ADN).

De telles amorces d'ADN ou d'ARN sont constituées d'environ 8 à 25 nucléotides identiques à une séquence de même longueur, de la séquence selon l'invention, ou complémentaires de ces nucléotides. D'autres amorces d'ADN ou d'ARN sont encore caractérisées en ce qu'il s'agit de fragments nucléotidiques capables d'hybrider avec les séquences selon l'invention dans des conditions 6xSSC, 2,5% lait, 100µg/ml DNA harreng 65°C, lavages en 6xSSC, 0,5xSSC.

Un fragment utilisable en tant qu'amorce est caractérisé en ce qu'il est susceptible d'induire la synthèse d'un acide nucléique complémentaire de celui auquel il s'hybride (ce dernier est alors appelé matrice) au sein d'une solution tamponnée et dans des conditions de pH et de température appropriées pour cette synthèse, en présence d'un agent inducteur de la synthèse, notamment une polymérase ou une réverse transcriptase et en présence des nucléotides appropriés. Le produit de synthèse obtenu à partir du fragment servant d'amorce est appelé produit d'allongement de l'amorce.

Des solutions tamponnées et conditions de pH et de température appropriées à la production du "produit d'allongement de l'amorce" peuvent être déterminées par référence aux enseignements des brevets américains précités dont le contenu est incorporé dans la présente demande.

Pour la réalisation de l'amplification en chaîne de l'ADN, on choisira des amorces d'ADN ou d'ARN complémentaires des deux extrémités de la séquence dont on recherche l'amplification. De préférence ces amorces sont situées de chaque côté de l'enchaînement répété de nucléotides qui a été présenté plus haut.

Les amorces précédentes peuvent être utilisées soit pour la préparation des séquences nucléotidiques de l'invention et le cas échéant pour la préparation du polypeptide pour lequel elle code ou encore, pour la réalisation de la détection in vitro de la présence de séquences nucléotidiques de P falciparum selon l'invention dans un échantillon biologique chez un patient susceptible d'être infecté par cet agent.

L'invention concerne donc un procédé pour l'amplification d'une séquence nucléotidique déterminée, comprenant les étapes suivantes,

a/ la mise en contact, en présence de nucléosides triphosphates et d'un agent inducteur de polymérisation, de l'échantillon biologique suspecté de contenir l'acide nucléique recherché, préalablement rendu accessible à une première amorce dans des conditions autorisant l'hybridation entre ces amorces et cet acide nucléique recherché, et la polymérisation à partir de ces amorces hybridées d'acide nucléique complémentaire pour produire un duplex formé entre le produit d'allongement de l'amorce, hybridé avec le brin d'acide nucléique recherché,

b/ la dénaturation du duplex obtenu à l'étape a/ de façon à "séparer" le produit d'allongement de l'amorce de l'acide nucléique à détecter,

c/ la mise en contact du produit d'allongement obtenu avec une deuxième amorce (au sens que l'on a donné plus haut de cette expression) ayant une séquence de nucléotides (1) non complémentaire de celle de la première amorce et (2) complémentaire d'une séquence du produit d'allongement précédemment formé,

d/ le cas échéant la répétition des étapes a/ et b/ et c/ en présence des première et deuxième amorces utilisées en excès et des réactifs nécessaires à la production de nouveaux produits d'allongement utilisés à leur tour comme matrice pour obtenir des synthèses supplémentaires, jusqu'à obtenir une quantité suffisante pour être détectée de produits d'allongement des amorces utilisées,

e/ la détection de la présence des produits d'allongement caractéristiques de la présence de l'ADN ou de l'ARNm de P falciparum.

Lorsque le nombre d'étapes d'amplification est jugé suffisant, on peut réaliser la détection de la séquence recherchée en utilisant un nucléotide radioactif.

Lorsque la métode d'amplification par la technique PCR est réalisée en vue de la production d'un antigène selon l'invention, on introduit les séquences nucléotidiques amplifiées dans un vecteur approprié, puis on réalise une culture dans un milieu de culture approprié d'un hôte cellulaire préalablement transformé par le susdit vecteur contenant l'acide nucléique amplifié, et on récupère à partir du milieu de culture, l'antigène produit par l'hôte cellulaire.

L'hôte cellulaire peut être choisi de façon à ce que l'antigène soit exprimé dans le cytoplasme. Dans ce cas on récupère l'antigène par lyse de l'hôte cellulaire. Selon une méthode avantageuse, on peut choisir l'hôte cellulaire et les éléments de régulation du vecteur recombinant contenant la séquence à exprimer, de façon à ce que l'antigène produit soit excrété à sa surface ou sécrété dans le milieu. L'antigène est ensuite purifié à partir de ce milieu.

Les peptides selon l'invention peuvent encore être préparés par les techniques classiques, dans le domaine de la synthèse des peptides. Cette synthèse peut être réalisée en solution homogène ou en phase solide.

Par exemple, on aura recours à la technique de synthèse en solution homogène décrite par HOUBENWEYL dans l'ouvrage intitulé "Methode der Organischen Chemie" (Méthode de la Chimie Organique) édité par E. Wunsch, vol. 15-I et II., THIEME, Stuttgart 1974.

Cette méthode de synthèse consiste à condenser successivement deux-à-deux les aminoacyles successifs dans l'ordre requis, ou à condenser des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonction amine de l'un et carboxyle de l'autre ou vice-versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthyl-aminopropyl)-carbodiimide.

Lorsque l'aminoacyle mis en oeuvre possède une fonction acide supplémentaire (notamment dans le cas de l'acide glutamique), ces fonctions seront protégées, par exemple par des groupes t-butylester.

Dans le cas de la synthèse progressive, acide aminé par acide aminé, la synthèse débute de préférence par la condensation de l'amino-acide C-terminal avec l'aminoacide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptide synthesis" (J. Am. Chem. Soc., 45, 2149-2154).

Pour fabriquer une chaîne peptidique selon le procédé de MERRIFIELD, on a recours à une résine polymère très poreuse, sur laquelle on fixe le premier acide aminé C-terminal de la chaîne. Cet acide aminé est fixé sur la résine par l'intermédiaire de son groupe carboxylique et sa fonction amine est protégée, par exemple par le groupe t-butyloxycarbonyle.

Lorsque le premier acide aminé C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième acide aminé qui fournit le second amino-acyle de la séquence recherchée, à partir du résidu amino-acyle C-terminal sur la fonction amine déprotégée du premier acide aminé C-terminal fixé sur la chaîne. De préférence, la fonction carboxyle de ce deuxième acide aminé est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux acide aminés, et dont la fonction amine terminale est protégée. Comme précédemment, on déprotège la fonction amine et on peut ensuite procéder à la fixation du troisième aminoacyle, dans les conditions analogues à celles de l'addition du deuxième acide aminé C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents acides aminés constituant la chaîne peptidique et on détache le peptide de la résine par exemple à l'aide d'acide fluorydrique.

De même, les acides nucléiques de l'invention peuvent être préparés soit par un procédé chimique, soit par d'autres procédés.

Un mode de préparation approprié des acides nucléiques comportant au maximum 200 nucléotides (ou 200 pb, lorsqu'il s'agit d'acides nucléiques bicaténaires) de l'invention comprend les étapes suivantes :

- la synthèse d'ADN en utilisant la méthode automatisée des β-cyanethylphosphoramidite décrite dans Bioorganic Chemistry 4; 274-325 (1986),

- le clonage des acides nucléiques ainsi obtenus dans un vecteur approprié et la récupération de l'acide nucléique par hybridation avec une sonde appropriée.

Un mode de préparation, par voie chimique, d'acides nucléiques de longueur supérieure à 200 nucléotides (ou 200pb, lorsqu'il s'agit d'acides nucléiques bicaténaires) de l'invention comprend les étapes suivantes :

- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Natl. Acad. Sci. USA, 80; 7461-7465, (1983),
- le clonage des acides nucléiques ainsi obtenus dans un vecteur approprié et la récupération de l'acide nucléique recherché par hybridation avec une sonde appropriée.

L'invention concerne également un procédé pour le suivi de la vaccination de patient contre l'infection contre P falciparum, à partir d'un échantillon biologique tel que le sang, caractérisé en ce qu'il comprend :

- la mise en contact de l'échantillon biologique susceptible de contenir des anticorps protecteurs contre P falciparum, avec au moins un antigène selon l'invention,
- la détection de la réaction antigène-anticorps.

Pour la réalisation de cette méthode de détection in vitro, les antigènes selon l'invention sont avantageusement marqués à l'aide d'un marqueur radioactif, d'un marqueur enzymatique ou fluorescent ou encore d'un marqueur de type marqueur physique.

L'invention vise également des kits pour la détection in vitro de la présence d'anticorps dirigés contre les antigènes de l'invention, caractérisés en ce qu'ils contiennent :

- une composition antigénique comprenant au moins un antigène selon l'invention,
- des réactifs nécessaires à la réalisation de la réaction immunologique entre les susdits antigènes et les anticorps éventuellement pris dans l'échantillon biologique,
- des réactifs permettant la détection du complexe antigène-anticorps produit par la réaction immunologique.

Ces réactifs sont par exemple porteurs d'un marqueur ou susceptibles d'être reconnus par un réactif marqué.

L'invention vise par ailleurs des anticorps dirigés contre les antigènes répondant aux définitions ci-dessus, ces anticorps étant polyclonaux ou monoclonaux.

Les anticorps monoclonaux peuvent être produits par la technique des hybridomes, suivant les techniques classiques comprenant :

- la fusion d'une cellule myelomateuse avec des cellules spléniques d'un animal préalablement immunisé avec l'un des antigènes selon l'invention,
- la culture des hybridomes formés par la fusion des cellules précédentes et,
- la sélection de ceux des hybridomes capables de former des anticorps monoclonaux reconnaissant l'antigène utilisé pour l'immunisation des animaux.

Les animaux choisis pour l'immunisation peuvent être par exemple des souris.

Parmi ces anticorps monoclonaux, on sélectionnera avantageusement les anticorps monoclonaux cytophiles, c'est à dire ceux dont le fragment Fc est capable de se fixer sur le récepteur Fc des monocytes humains.

Une autre technique de production d'anticorps peut permettre de réaliser in vitro des anticorps monoclonaux humains. Pour ce faire, on utilise des lymphocytes B immortalisés par exemple avec le virus d'Epstein Barr. Ces lymphocytes peuvent être prélevés chez une personne ayant été infectée par P falciparum. Dans ce cas, ils permettent la production d'anticorps monoclonaux contre plusieurs antigènes sans avoir recours à une stimulation in vitro par de nouveaux antigènes.

Une autre possibilité consiste à fusionner des lymphocytes B immortalisés de la façon décrite plus haut, avec des lymphocytes B humains préalablement stimulés in vitro avec un antigène selon l'invention contre lequel on cherche à former des anticorps monoclonaux, dans des conditions de culture permettant la stimulation des lymphocytes.

On se reportera avantageusement à la technique décrite par Desgranges C. et al (1987, J of Virological Methods, vol 16, p281-292) pour la préparation des anticorps monoclonaux humains de l'invention.

Il est également envisagé dans le cadre de l'invention d'humaniser des anticorps monoclonaux obtenus chez l'animal en réalisant une transfection in vitro du gène codant pour la partie variable de l'anticorps, dans des cellules humaines, dans des conditions permettant la fixation d'un fragment constant Fc humain à cette partie variable.

Des anticorps monoclonaux préférés selon l'invention sont des anticorps humains de classe IgG$_1$ ou IgG$_3$, ou des anticorps obtenus chez l'animal et ayant des propriétés cytophiles chez l'homme, dirigés contre l'antigène R23 dont la séquence a été décrite plus haut.

D'autres avantages de l'invention apparaissent dans les exemples qui suivent.

**Figure 1** : carte de restriction de la séquence codant pour l'antigène P falciparum

**Figure 2** : séquence nucléotidique de l'antigène de P falciparum

**Figure 3** : séquence d'acides aminés de l'antigène de P falciparum

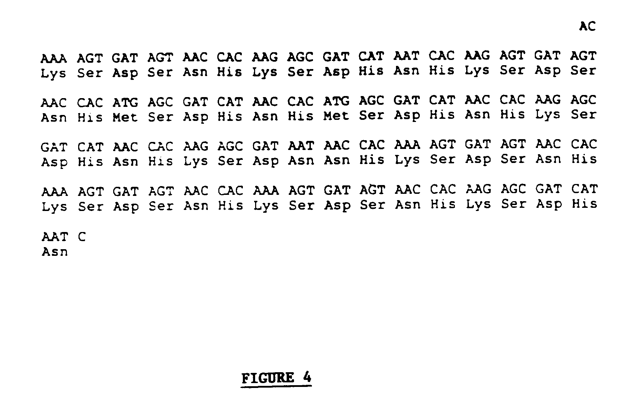

**Figure 4** : clone R23, séquence nucléotidique et sa séquence en acides aminés correspondante

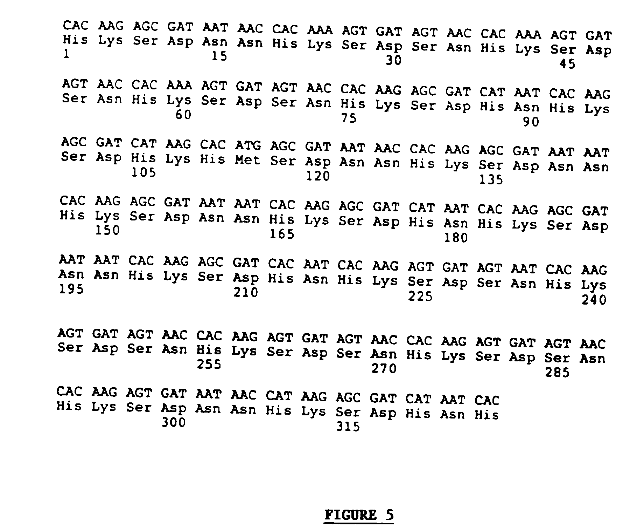

**Figure 5** : clone R45, séquence nucléotidique et sa séquence en acides aminés correspondante

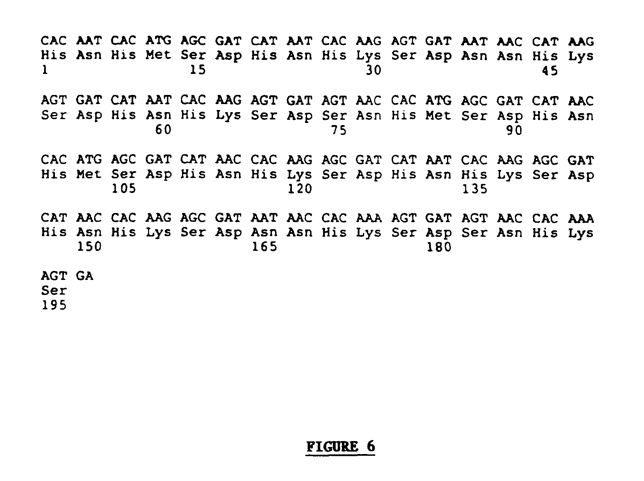

**Figure 6** : clone R51, séquence nucléotidique et sa séquence en acides aminés correspondante

**Figure 7** : influence de la concentration d'antigène sur l'intensité du signal ELISA

Des gammes de concentration d'antigène GT, GTR45, ou GTHRPII, ont été préparées. Les plaques ont été ensuite incubées avec plusieurs antisérums. La fixation des anticorps est mise en évidence par la liaison d'anti-immunoglobulines marquées à la peroxydase. L'activité enzymatique révélée est proportionnelle à la quantité d'anticorps fixés sur ces plaques.

A. plaques chargées en antigène GTR45, incubées avec un antisérum de souris anti-GT (●...●) ou l'antisérum du lapin 311, immunisé avec la β-gal R23 (O...O);
B. plaques chargées avec l'antigène GTHRPII, utilisé comme contrôle, incubées avec un antisérum de souris anti-GT (▲...▲) ou avec un antisérum anti-HRPII (Δ ... Δ); C. plaques chargées avec l'antigène GT vecteur, incubées avec un antisérum de souris anti-GT (■...■).

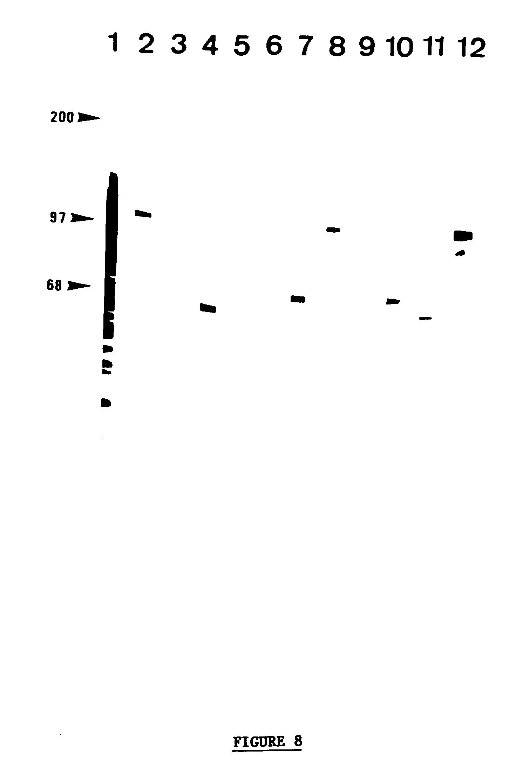

**Figure 8** : immunoblot de P falciparum révélé avec divers antisérums de souris immunisées. Les antisérums étaient :

    a) 1 et 12, anti B-gal H92
    b) anti extrait d'E.coli SB95
    2. souris BALB/c (groupe 1)
    3 à 6 (groupe 2), souris outbred
    3. souris 45-1 4. souris 45-2 5. souris 45-3 6. souris 45-4
    c) anti extrait d'E.coli lysogène pour lambda gtll R23 (groupe 3)
    7. souris 23-1 8. souris 23-2 9. souris 23-3
    10. souris 23-4 11. souris 23-5

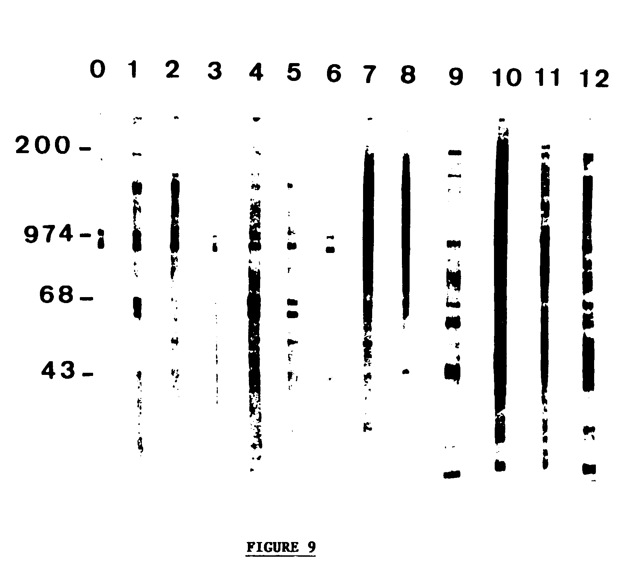

**Figure 9** : immunoblot de P falciparum Palo Alto Marburg révélé avec divers antisérums

    O. sérum de souris non immune
    1. sérum de la souris 23-1
    2. sérum de la souris 23-2
    3. sérum de la souris 23-3
    4. sérum de la souris 23-4
    5. sérum de la souris 23-5
    6. sérum préimmun de souris BALB/c immunisées avec l'extrait brut de ,la lysogène pour lambda gt11R23
    7. sérum des souris précédentes, après immunisation
    8. sérum de la souris A2
    9. sérum préimmun du lapin 311
    10. sérum immun du lapin 311
    11. sérum préimmun du lapin 310
    12. sérum immun du lapin 310

## EXEMPLES

### I - __Isolement de clones R23, R45, (et R51)__

a) Construction de la banque

Une banque de DNA (ADN) génomique a été construite dans le bactériophage d'expression λgt11, en utilisant l'ADN génomique du clone Tak 9-96 de P falciparum (ref clone Tak 9-96 : Science 212, 137; 1981) suivant le protocole décrit en détail dans la demande de brevet EP du 9 février 1987 publiée sous le numéro 0343186.

En bref, l'ADN a été découpé par la DNAase I, en présence d'ions Mn2+, méthylé par la méthylase EcoRI pour protéger les sites EcoRI naturels, puis réparé par l'ADN polymérase du bactériophage T4 et l'ADN ligase d'EcoRI. Des "linkers" oligomères synthétiques) EcoRI ont été ligaturés aux fragments de DNA de P falciparum et les sites artificiels ainsi rajoutés ont été libérés par coupure avec l'enzyme EcoRI. Les fragments ont été purifiés sur gradient de sucrose et ligaturés avec le DNA du vecteur λgt11 convenablement préparé (c'est à dire coupé par EcoRI et déphosphorylé - vendu par Promega Biotec). L'ADN a été encapsidé in vitro dans des particules virales. Les bactériophages issus de ce procédé constituent une banque d'ADN génomique.

b) Criblage de la banque

Les détails techniques du criblage sont donnés dans le texte de la demande de brevet 0343186. Le sérum utilisé était celui du Lapin R96. Ce lapin a été immunisé avec une préparation antigénique constituée des protéines migrant dans la région 100kDa, préparée par électroélution de gels préparatifs de polyacrylamide SDS chargés avec le surnageant de culture de formes très mûres de P falciparum (surnageant chauffé à 100°C 15 min). L'immunisation a eu lieu avec comme adjuvant le poly A/poly U. Le sérum a été épuisé en anticorps anti E.coli avant d'être utilisé pour cribler la banque.

En bref on prépare des plages de lyse de bactériophages de la banque (environ 5 à 10.000 plages par boite). On cultive 3H à 37C. On ajoute alors un filtre de nitrocellulose saturé en IPTG $10^{-2}$M, on fait des repères sur la boite et le filtre et l'on prélève le filtre que l'on sature puis on l'incube avec des anticorps. La révélation des complexes Ag/Ac est effectuée par la protéine $A^{125}I$. Après lavage on autoradiographie les filtres. On positionne le filtre sur les boites, ou on prélève la région et on réétale pour réaliser un criblage secondaire en une série de 3-4 criblages successifs. On enrichit et on purifie les bactériophages produisant un antigène reconnu par le sérum et on termine avec un clone pur de bactériophage.

c) Lysogénisation

Le phage pur est utilisé pour infecter la souche Y1089 (Young et Danies, Science, 222, 778; 1983).

Les lysogènes sont purifiées, on extrait l'ADN du bactériophage, on coupe par EcoRI pour libérer l'insertion d'ADN génomique de P falciparum véhiculée par le bactériophage en question (lysogène déposée pour le bactériophage λ R45 = SB95, I-985, 27.07.90).

d) Reclonage dans d'autres vecteurs

L'ADN du bactériophage découpé est recloné dans le vecteur pMS1 coupé par ECORI, déphosphorylé : on mélange l'ADN du vecteur et l'ADN du bactériophage R23 coupé par EcoRI avec de la T4 DNA ligase. On transforme ensuite des bactéries hôtes rendues compétentes à l'introduction d'ADN et on sélectionne les colonies ampiciline-R (résistantes à l'ampiciline). On effectue un criblage immunologique pour rechercher les colonies qui ont inséré le fragment R23.

Les clones produisent une β-galactosidase enzymatiquement active que l'on purifie sur colonne d'APTG-Sépharose (clone SB275).

e) Obtention de la séquence d'ADN

L'insertion est préparée comme précédemment, mais reclonée dans le bactériophage M13mp18 ou M13mp19. Les bactériophages recombinants sont repérés par hybridation à l'insertion pure, et l'ADN est préparé et séquence selon la technique Sanger.

f) Clonage de grands fragments (par exemple : DdeI)

On établit une carte de restriction de la région génomique portant les séquences hybridant par exemple à R45 et on choisit une coupure ou un type de coupure qui produit des fragments dont on espère qu'ils contiendront tout le gène. Ici on a choisi DdeI : on digère le DNA génomique souche Palo Alto par DdeI, on électroélue les fragments migrant dans la zone de PM 4 à 6 Kb. Ensuite ces fragments sont réparés par la T4 DNA polymérase, des linkers EcoRI sont additionnés et le tout est ligaturé avec le bactériophage λgt WES coupé par EcoRI. Le criblage est fait par hybridation avec l'insertion du clone R45. A chaque étape de criblage, on éliminera manuellement (par ponction avec une pipette Pasteur) les bactériophages qui forment des grosses plages. En quatre séries successives d'amplification, on a obtenu suffisamment de bactériophages pour préparer de l'ADN, extraire l'insertion par EcoRI et recloner dans le vecteur puc13 coupé par EcoRI et la phosphatase (Pharmacia). Après ligation avec ce vecteur, l'ADN est introduit :

soit dans DH5 α

soit dans CMK603 dans lequel il forme la souche SG3636 déposée sous le numéro I-987

II - Propriétés de la βgalactosidase R23 (βgal-R23)

La βgal recombinante exprimée par le clone R23 peut être purifiée à partir des extraits solubles d'E.coli n° 275 après induction par l'IPTG. Ces bactéries hébergent un plasmide du type pMS (Scherf et al, 1990, J. Immunol Methods, 128 : 81) dans lequel a été inséré le fragment d'ADN génomique parasitaire excisé par l'enzyme EcoRI à partir de l'ADN du bactériophage recombinant R23. Ce plasmide pMS23 code pour une protéine de fusion βgal-R23 enzymatiquement active, qui eut être purifiée sur une colonne d'affinité du type APTG-Sépharose.

Avec cet antigène recombinant on peut préparer des colonnes d'affinité en immobilisant l'antigène sur la Sépharose CH activée (Pharmacia), selon le protocole décrit par Pharmacia. On retient alors sur cette colonne R23 les anticorps spécifiques de la séquence R23 (et, si l'on a pas pris soin de les éliminer avant les anticorps anti-β-galactosidase).

Le passage sur cette colonne R23 diminue considérablement la capacité de certains sérums humains immuns de promouvoir une activité de type ADCI sur P falciparum en culture.

Absorption du sérum sur protéine recombinante βgal-R23 immobilisée

400 µl de sérum SHI2 (Sérum Humain Hyperimmun n°2) sont incubés avec 300 µl de billes (en PBS), avec agitation douce. L'incubation est effectuée pendant 1 heure à température ambiante. Le surnageant est récupéré puis dialysé en RPMI 1640 et testé en ADCI.

Index spécifique d'inhibition

| | |
|---|---|
| SHI2 non traité | 70% |
| SHI2/βgal | 63% |
| SHI2/βgal-R23 | 34% |

Cette expérience d'ADCI montre que lorsqu'on cultive en présence de sérum humain hyperimmun et de monocytes, on a au bout de 48 heures une inhibition de 70% de la culture (au lieu de 5% de parasitémie on a 1,5%). Quand on absorbe ce sérum sur une colonne de sépharose couplé à la βgalactosidase il y a encore une inhibition importante (63%), alors que quand on passe le sérum sur une colonne où on a immobilisé la βgalactosidaseR23, on perd des anticorps qui interviennent dans l'ADCI et l'inhibition n'est plus que de 34%.

Cette expérience montre que, après absorption des anticorps anti-R23, on élimine la spécificité R23. On le vérifie par la perte de réactivité sur des dots blots de βgal-R23 alors que la réactivité sur βgal-H92 est intacte.

Une deuxième expérience a été réalisée : quand on prend un pool d'IgG hyperimmunes (pool d'immunoglobulines humaines immunes, protectrices en transfert passif chez l'homme) on a 50% d'inhibition; quand on rajoute le sérum de la souris (23-2), on lève toute inhibition. Les IgG de souris entrent en compétition pour l'antigène mais ne sont pas capables de stimuler les monocytes humains. Les contrôles de sérum indiquent que les sérums de souris n'inhibent pas la culture par eux-mêmes (sérum anti-R45), et sont mêmes facilitants (cas des sérums anti-R23 et anti-H92).

Pool IgG hyperimmuns (PIAG)

| PIAG (2,5 mg/ml) | 50% |
|---|---|
| souris anti-R23 2 (1/100) | - 10% |
| souris anti-H92 (1/100) | - 5% |
| souris anti-R45 (BALB/C) (1/100) | 0 |
| PIAG + anti-R23 | 0 |
| PIAG + anti-H92 | 40% |
| PIAG + anti-R45 | 30% |

Il est aussi possible de faire des compétitions avec des sérums d'enfants par exemple. Ces sérums reconnaissent l'antigène R45/R23 mais avec des sous-classes $IgG_2$ et $IgG_4$ incapables de coopérer avec les monocytes.

III- Etude de la réponse contre les motifs répétés présentés par les insertions R23 et R45 : réponse humorale d'individus exposés au paludisme en zone d'endémie (Madagascar et Sénégal)

La réponse humorale contre les motifs répétitifs en utilisant l'antigène recombinant GT R45 ou la β-galactosidase R23 a été évaluée.

Des essais préliminaires ont été réalisés pour évaluer le meilleur antigène à employer pour doser les anticorps de sujets africains. Les mises au point et les résultats des dosages sont décrits plus bas.

De très nombreux sérums humains comprennent des anticorps reconnaissant les protéines-vecteurs comme la glutathione transférase du vecteur pGEX ou la β-galactosidase. La réponse des divers individus au protéines recombinantes comportant la GT ou la β-galactosidase a donc été appréciée en tenant compte du bruit de fond sur le vecteur.

La situation épidémologique la mieux explorée est celle où est effectuée une enquête épidémologique au sénégal, dans le village de Dielmo. On observe une situation assez tranchée : les titres élevés en anticorps ne sont trouvés que chez les personnes de plus de 7 ans et surtout au delà de 15 ans, âge auquel se met en place une immunité assez efficace. Il demeure qu'une proportion non négligeable de la population ne semble pas produire d'anticorps de façon significative (40 à 50% chez les enfants, 9 à 30% chez les adultes). Ces données doivent être nuancées en raison du signal détecté sur le vecteur qui doit être pris en compte car il peut masquer des positivités faibles.

Les résultats obtenus indiquent que la spécificité R23-R45 peut être la cible d'anticorps intervenant par le mécanisme d'ADCI. Une des prédictions que l'on peut faire s'il s'agit d'un mécanisme important est que non seulement le titre en anticorps anti R23-R45 est un facteur important de la protection, mais que la sous classe des immunoglobulines intervient aussi dans la protection conférée. Les immunoglobulines de classe cytophile ($IgG_1$ et $IgG_3$ chez l'homme) sont les plus opérationnelles car les autres isotypes risquent de rentrer en compétition pour la fixation à l'antigène empêchant ainsi le déclenchement de l'ADCI. Des résultats obtenus sur les sérums collectés à Dielmo montrent qu'il y a de fait déséquilibre de sous-classe chez les adultes analysés (voir plus bas).

IV - Identification de la protéine parasitaire codée par le gène R45

Une analyse de la production d'ARN messager correspondant au gène R45 au cours du cycle cellulaire asexué, a permis de mettre en évidence que le gène est exprimé essentiellement au stade trophozoite jeune. La présence d'une protéine de 160kDa est corrélée avec celle de l'ARNm.

V - Mise au point des conditions de dosage des anticorps humains reconnaissant les épitopes R45/R23

Du fait des propriétés intéressantes de la séquence protéique R23/R45 dans l'ADCI, ce qui a été tenté est de mesurer les anticorps de cette spéficité.

Le peptide synthétique HKSDNNHKSDSNHKSDSN a été utilisé pour charger des puits de plaques ELISA. Le peptide est reconnu spécifiquement par plusieurs sérums humains, mais n'est pas reconnu - sur ces plaques ELISA - par plusieurs autres anticorps monoclonaux qui pourtant reconnaissent spécifiquement les protéines recombinantes β-galR23, MS2PolyméraseR45 ou GTR45. On peut dire en première approximation que le peptide se fixe au support (plastique des plaques ELISA ou nitrocellulose) par un de ses déterminants antigéniques. Les résultats de l'invention indiquent que ce peptide contient au moins deux épitopes, puisque sur les plaques ELISA certains sérums humains le reconnaissent.

De plus, le peptide synthétique ne présente pas tous les épitopes portés par la molécule R23 ou R45. Lorsqu'on incube un sérum humain sur la colonne de peptide Sépharose de façon à éliminer tous les anticorps reconnaissant le peptide, il reste néanmoins des anticorps susceptibles de se fixer aux protéines β-galR23 et GTR45. Ceci indique que les 11 répétitions portées par la β-galR23 et les 17 répétitions portées par la GTR45 contiennent des déterminants antigéniques qui ne sont pas présentés par ce peptide synthétique.

Donc le peptide synthétique permet une analyse partielle de la réponse humaine en anticorps aux répétitions R45/R23, lorsque le test de détection des anticorps fait appel à la fixation de l'antigène.

### 1) Utilisation du fragment R23 purifié

On peut séparer le fragment parasitaire R23 de la β-galactosidase-vecteur en coupant la molécule recombinante par la protéase facteur Xa. Cette protéase reconnait une séquence d'acides aminés introduite entre la β-galactosidase et l'insertion parasitaire par le vecteur pMS. Une fois la coupure opérée, on sépare sur gel de polyacrylamide les deux éléments, le fragment R23 migrant plus rapidement que la β-galactosidase. Dans ces conditions aussi, on observe que les anticorps monoclonaux sont incapables de se fixer aux séquences R23. Ceci indique que le fragment parasitaire de 11 répétitions se fixe à la colonne d'électrophorèse par un déterminant antigénique lorsqu'il n'est plus fusionné à une protéine porteuse. La fixation semble se faire de façon très monotone ou homogène, probablement par une ou des chaînes latérales essentielles à l'un des déterminants antigéniques.

### 2) Dosage au moyen de molécules recombinantes

Un premier choix a donc porté sur l'emploi de molécules recombinantes intactes. Il faut alors tenir compte de la présence dans les sérums humains de taux variables d'anticorps susceptibles de se fixer à la protéine vectrice, β-galactosidase ou GT. Les signaux obtenus avec la β-galactosidase vecteur semblant être plus importants que ceux observés avec la GT dans les sérums africains, l'emploi de molécules recombinantes GT a été décidé.

Les résultats indiquent que les propriétés antigéniques de la protéine R45 sont très proches de celles de la protéine R23. Les ELISA pratiqués sur des plaques chargées en antigène GTR45 (GT représente la glutathione transférase de Schistosoma japonicum codée par le vecteur décrit par Smith D.B & Johnson K.S, 1988, Gene <u>67</u>: 31) ou bien en GTR23 montrent que les valeurs obtenues sont voisines, celles sur GTR23 étant légèrement plus faibles que celles sur GTR45.

La détermination de la liaison sur la protéine recombinante GTR45 et celle sur le vecteur GT permet d'estimer les anticorps anti R45 par la formule

$$\text{DO R45} = \text{DO GT R45} - \text{DO GT}$$

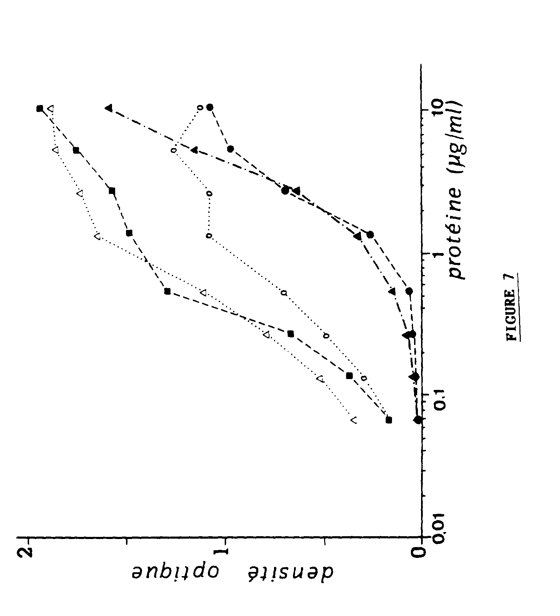

Cette mesure sous-estime les anticorps anti R45. En effet, la valeur mesurée sur GT vecteur est surestimée comme le montre la figure 7. Dans cette expérience on a fait varier la concentration de l'antigène utilisé pour charger les plaques, GT (vecteur), GTR45 ou GTHRPII. HRPII a été décrite par Knapp B et al, (Behring Res. Comm. <u>B2</u>: 349-359). On a ensuite incubé avec un antisérum contre R23 ou contre HRPII (courbes aux symboles ouverts et en pointillés). Ces deux courbes indiquent que les protéines se fixent sur les plaques et que le plateau de concentration est atteint aux alentours de 1 µg/ml. Par contre, lorsqu'on incube avec un sérum de souris immunisée contre GT (ce sont les courbes aux symboles pleins), on observe des courbes très différentes : les deux protéines recombinantes sont alors très mal reconnues. Et surtout, pour une concentration molaire équivalente, on a un signal au moins dix fois plus fort sur GT vecteur que sur GTR45 ou GTHRPII (les rapports molaires sont tels que l'on a le même nombre de molécules GT dans 0,7 µg de GT que dans 1 µg de GTR45 ou GTHRPII).

Ceci montre que les protéines GTR45 ou GTHRPII fixent les anticorps anti-GT beaucoup moins bien que la protéine GT vecteur. En fait le symbole "GT" ne représente pas la même réalité immunologique dans GT-vecteur et dans GT-recombinant. Avec les concentrations utilisées la surestimation de la réponse due au vecteur peut aller jusqu'à un facteur 10.

Il est clair que si l'on détermine les anticorps anti "GT" au moyen de GT-vecteur, on aura des valeurs beaucoup plus élevées que si on les détermine au moyen d'un GT-recombinant non apparenté.

Par conséquent l'évaluation des résultats obtenus à l'aide la formule ci-dessus doit être modulée au niveau du résultat DO GT. En d'autres termes, le taux d'anticorps anti-R45 (DO R45) présents chez une personne est supérieur au résultat calculé à l'aide de la formule ci-dessus.

### VI- Résultats de la sérologie du village de Dielmo (Senégal)

Les anticorps ont été mesurés selon la technique ELISA décrite ci-dessous. Les antigènes utilisés pour charger les plaques étaient GT vecteur, GTR45, GTH92 et GTHRPII. H92 a été décrite par Bonnefoy S. et al (Experimental Parasitology 65: 69-83 (1988)).

Technique ELISA

L'antigène est purifié selon la technique décrite par Smith et Johnson (ibidem). Il est dilué dans le tampon PBS (150mM NaCl; 10mM tampon phosphate; pH 7,4). Les plaques (de la marque CML) sont chargées avec 50µl par puits de l'antigène. Les antigènes recombinants sont à 1µg/ml et le GT vecteur est à 0,7 µg/ml. Le "coating" est effectué toute la nuit à 4°C. Après aspiration des solutions d'antigène, les plaques sont saturées avec le tampon PBS, 0,05% Tweens®, 20,5% lait écrémé Régilait pendant 1 heure à 37°C; les antisérums sont dilués au centième en PBS, 0,05% Twen20® (PBS T) et incubés à raison de 100µl par puits pendant 1 heure à 37°C. Après lavages, on dépose dans les puits 100µl d'une solution d'anti IgG, A, M humaines marquées à la peroxydase (Biosys), diluées au 1/4000 en PBS T; les plaques sont incubées à 37°C 1 heure, lavées et la fixation de la peroxydase est révélée par le réactif TMB (kpl).

Dans 182/202 cas, la valeur obtenue sur GTR45 est plus élevée que celle obtenue sur GT vecteur. Les taux de positivité pour H92 et HRPII sont respectivement de 49/202 et 32/202.

Dans 20 cas, soit 10% de l'échantillon, cette valeur est plus faible. Pour GTH92, c'est 97 cas sur 202 qui ont des valeurs inférieures à celles obtenues sur GT, soit 48%; pour GTHRPII, c'est 40% des dosages qui sont plus faibles sur le vecteur que sur la protéine recombinante.

Il y a donc 90% des sérums qui donnent un signal positif sur les répétitions de R45. Ces résultats indiquent une très forte prévalence de positivité.

Si on soustrait la valeur lue sur GT vecteur de la valeur observée sur GTR45, on obtient une répartition par tranche d'age indiquée au Tableau I.

Les enfants présentent des positivités faibles. Aucun des enfants (dont nous avons du doser les anticorps) de moins de 6 ans n'est dans la classe des fortes réponses. Dans les tranches d'âge en dessous de 13-14 ans, d'une manière générale les réponses sont assez basses. A partir de 15 ans, par contre, à l'âge où s'installe une immunité anti parasitaire efficace, on observe des réponses fortes. Environ 30% de la population est classée comme faiblement positive et de 15 à 44% sont retrouvés dans la classe de très forte réponse.

L'appréciation des sous-classes d'IgG intervenant dans la liaison avec R45 a ensuite été faite. Des plaques ELISA ont été chargées en antigène soit GTR45 soit GT vecteur, incubées avec les antisérums. Puis, on a déposé des anticorps monoclonaux spécifiques de chaque sous-classe humaine, $IgG_1$, $IgG_2$, $IgG_3$ ou $IgG_4$. La dilution de ces anticorps est telle que des valeurs ELISA comparables reflètent des quantités comparables d'IgG. La fixation des anticorps est ensuite visualisée après incubation avec des anti-IgG de souris marquées à la peroxydase. La détermination des sous-classes a été réalisée seulement sur les plus forts répondeurs.

Sur 31 dosages, la répartition est la suivante :

| | |
|---|---|
| $IgG_2$ | |
| $IgG_1+IgG_2$ | 2 |
| $IgG_2+IgG_3$ | 1 |
| $IgG_1$ | 1 |
| $IgG_3$ | 13 |
| $IgG_1+IgG_3$ | 8 |
| $IgG_1+IgG_2+IgG_3$ | 2 |

soit 77% de réponses dans lesquelles il y a une répartition des sous-classes favorables aux sous-classes cytophiles.

**Répartition de la réponse contre les répétitions R45 à Dielmo (Sénégal) en fonction de l'âge**

| Age | 0-2 a | | 3-4 | | 5-6 | | 7-8 | | 9-10 | | 11-14 | | 15-20 | | 21-25 | | 26-30 | | 31-40 | | 41-50 | | >50 | |
|---|---|---|---|---|---|---|---|---|---|---|---|---|---|---|---|---|---|---|---|---|---|---|---|---|
| ELISA Valeur → | N | % | N | % | N | % | N | % | N | % | N | % | N | % | N | % | N | % | N | % | N | % | N | % |
| 0-200 | 5 | 55 | 6 | 40 | 6 | 40 | 8 | 61 | 8 | 50 | 7 | 46 | 8 | 30 | 3 | 21 | 6 | 31 | 6 | 27 | 2 | 11 | 2 | 8 |
| 201-400 | 2 | 22 | 6 | 40 | 6 | 40 | 3 | 25 | 6 | 375 | 2 | 13 | 8 | 30 | 4 | 28 | 5 | 26 | 7 | 32 | 5 | 27 | 7 | 9 |
| 400-600 | 2 | 22 | 3 | 20 | 3 | 20 | 0 | | 1 | 6 | 2 | 13 | 6 | 23 | 2 | 14 | 3 | 16 | 5 | 23 | 3 | 16 | 5 | 30 |
| > 600 | 0 | | 0 | | 0 | | 2 | 15 | 1 | 6 | 4 | 26 | 4 | 15 | 5 | 36 | 5 | 26 | 4 | 18 | 8 | 44 | 9 | 22 |
| n | 9 | | 15 | | 15 | | 13 | | 16 | | 15 | | 26 | | 14 | | 19 | | 22 | | 18 | | 23 | |

Déséquilibre net $\delta_1$ $\delta_3$ chez les forts répondeurs

VII- Réactions croisées immunologiques

Divers animaux ont été immunisés avec soit des extraits d'E.coli recombinant soit la β-galR23.

A. Immunisation avec l'extrait de la lysogène Y1089 (lambda gt11 R45) induite, souche déposée SB95 sous le numéro I-985.
Groupe 1 : souris BALB/c
Groupe 2 : souris outbred

B. Immunisation avec l'extrait de la lysogène Y1089 (lambda gt11 R23) induite
Groupe 3 : souris outbred

C. Immunisation avec la β-galR23 purifiée à partir d'extraits induits de la souche SB275 déposée sous le numéro I-986.

D. Immunisation de souris BALB/c avec la β-gal A2. Le fragment A2 ( environ 2078 pb ) qui correspond au segment qui va du site de restriction Ddel au site de restriction Alul immédiatement situé après les 80 répétitions dans l'insertion portée par le plasmide de la souche SB363 déposée sous le numéro I-987. La β-gal recombinante, de PM environ 200kDa a été purifiée à partir du culot de protéines insolubles en PBS, en remettant la protéine en solution en 6M urée.

Les divers animaux présentent des réponses très diverses en termes d'antigène parasitaire reconnu. Lorsqu'on analyse sur des immunoblots d'extraits parasitaires de Plasmodium falciparum, souche PALO ALTO, l'antigène parasitaire reconnu par les anticorps induits lors de l'immunisation, on observe des images différentes suivant les sérums. Ceci est illustré dans les figures 8 et 9. Dans la figure 8, les sérums des groupes 1, 2, et 3 sont analysés. Chaque souris individuelle présente une réponse particulière. Toutes ne reconaissent pas un antigène de PM 100kDa. Le groupe 1 a répondu en produisant des antigènes qui reconnaissent un antigène de 100kDa identifié comme étant probablement la 96 tR (colonne 2). Dans un groupe 2, on notera l'image obtenue avec le sérum de la souris n°2, colonne 4, qui se lie fortement à un groupe d'antigènes migrant vers 60kDa, et faiblement à un antigène de 100kDa et à un antigène de 160kDa. C'est le sérum de cette souris qui a bloqué l'effet ADCI du PIAG.

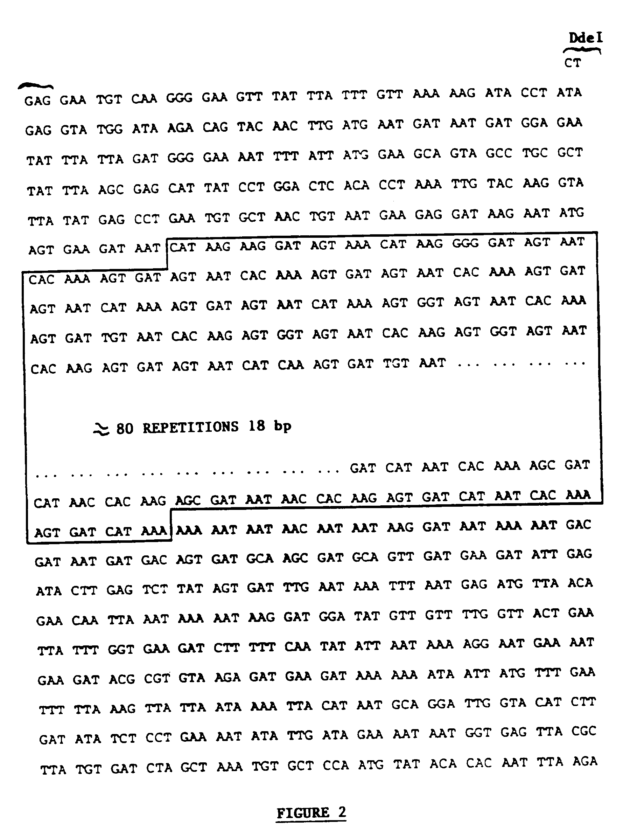

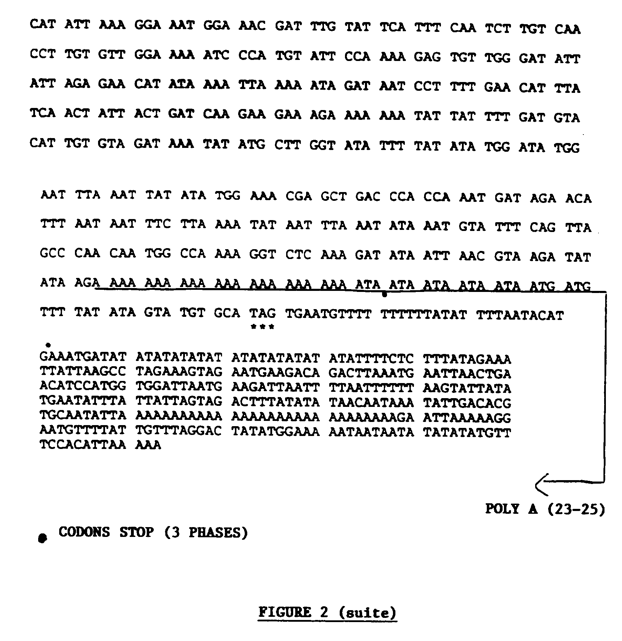

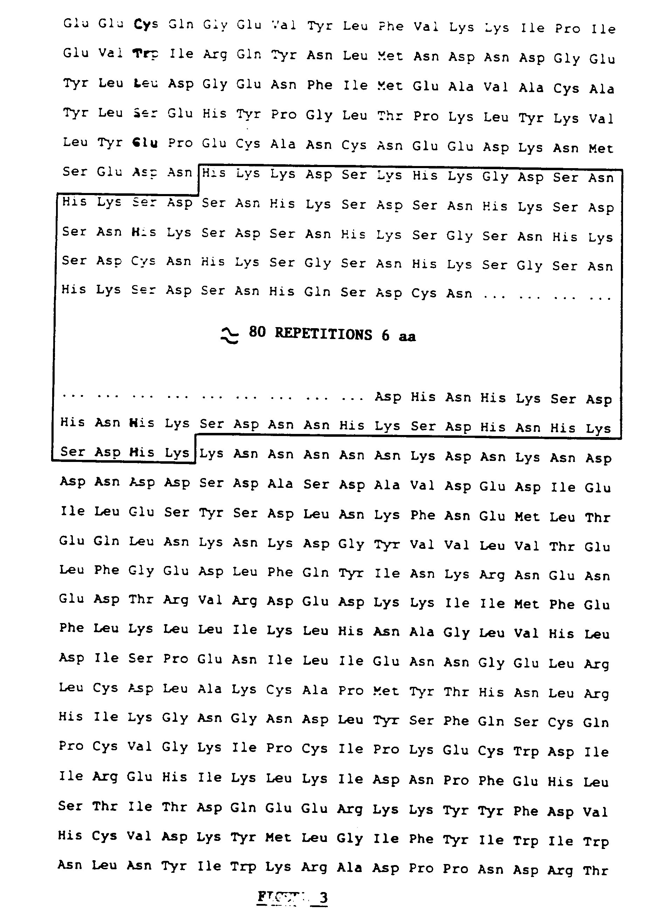

Dans la figure 9, ces mêmes sérums sont analysés sur un extrait de PALO ALTO Marburg. L'immunoblot était plus chargé en parasites et a permis de mettre en évidence la reconnaissance d'un antigène à peine visible sur le blot de la figure 2, migrant à 160kDa. La figure 9 montre aussi les réactions observées avec l'antisérum β-galA2 (colonne 8), ou celui du lapin 311 (colonne 10) qui eux aussi présentent une réaction très intense sur l'antigène de 160kDa. Le lapin 310 par contre n'a pas produit d'anticorps contre les antigènes de 100kDa (en fait on voit très clairement qu'il possédait avant immunisation des anticorps de cette spécificité), ou de 160kDa, mais a produit une réponse contre des antigènes migrant dans la région 60kDa (colonne 12).

Les résultats présentés indiquent très clairement que les répétitions R45 ou R23 sont immunogènes et que la réponse induite contre ces répétitions varie suivant les individus. Par ailleurs, on peut aussi conclure que des déterminants antigéniques proches sont présentés par d'autres antigènes parasitaires que l'antigène R45/R23 : le parasite possède des antigènes qui tissent un réseau complexe de réactions croisées. Une expérience où les ARN messagers produits à différents moments au cours du cycle érythrocytaire ont été hybridés avec la sonde R45 a montré que l'ARN hybridant avec cette sonde est produit par les stades jeunes. Le pic de synthèse de l'ARN et sa cinétique de produciton sont corrélés avec la présence de l'antigène de 160kDa.

VIII-Expérience de vaccination avec l'antigène GT R23

Une expérience de vaccination a été effectuée à l'Institut Pasteur de Cayenne. Des singes Saimiri sciueus ont été immunisés avec un mélange de 4 antigènes recombinants, parmi lesquels l'antigène GT R23. Les singes ont très bien résisté à l'épreuve de challenge par la souche FUP/S.

**Revendications**

1. Séquence nucléotidique de P falciparum, caractérisée en ce que :

- elle comprend un enchainement de nucléotides comportant n répétitions successives' de fragments nucléotidiques identiques ou non de 18 paires de bases (bp),lesditsfragmentsnucléotidiquescodant

    . pour des peptides identiques ou non, de 6 acides aminés répondant au motif de base suivant Asp b Asn His Lys Ser dans lequel b est un acide aminé quelconque, ou
    . pour une variante de ce motif de base obtenue par substitution d'un ou plusieurs acides aminés dès lors que les propriétés antigéniques et/ou immunogènes. de l'oligopeptide formé ne sont pas modifiées,

- n est suffisant pour que l'enchainement nucléotidique formé code pour un oligopeptide capable d'induire <u>in vivo</u> des anticorps qui reconnaissent l'antigène codé par la séquence nucléotidique de <u>P falciparum</u> répondant à la carte de restriction donnée ci-après :

GENE R45

A : Alu I
Ac : Acc I
R : Rsal
✳ : codon slop
Rp : = 7répétitions 45 bp

2. Séquence nucléotidique selon la revendication 1 caractérisée en ce que le motif de base est modifié et correspond à un peptide choisi parmi :

Asp Ser Asn His Met Ser

Asp Ser Asn His Met Ser

Ser Asn His Met Ser

Glu Ser Asn His Lys Ser

Asp Ser Asn His Glu Ser

3. Séquence nucléotidique selon la revendication 1 ou 2 caractérisée en ce que b est de préférence Asn, His, Ser, Cys.

4. Séquence nucléotidique de <u>Plasmodium falciparum</u> selon l'une quelconque des revendications 1 à 3, caractérisée en ce qu'elle comprend l'enchainement nucléotidique R23 répondant à la séquence suivante :

AC

AAA AGT GAT AGT AAC CAC AAG AGC GAT CAT AAT CAC AAG AGT GAT AGT

AAC CAC ATG AGC GAT CAT AAC CAC ATG AGC GAT CAT AAC CAC AAG AGC

GAT CAT AAC CAC AAG AGC GAT AAT AAC CAC AAA AGT GAT AGT AAC CAC

AAA AGT GAT AGT AAC CAC AAA AGT GAT AGT AAC CAC AAG AGC GAT CAT

AAT C

**5.** Séquence nucléotidique de <u>Plasmodium falciparum</u> selon l'une quelconque des revendications 1 à 3, caractérisée en ce qu'elle comprend l'enchainement nucléotidique R45 répondant à la séquence suivante :

```
CAC AAG AGC GAT AAT AAC CAC AAA AGT GAT AGT AAC CAC AAA AGT GAT
1               15              30              45

AGT AAC CAC AAA AGT GAT AGT AAC CAC AAG AGC GAT CAT AAT CAC AAG
        60              75              90

AGC GAT CAT AAG CAC ATG AGC GAT AAT AAC CAC AAG AGC GAT AAT AAT
        105             120             135

CAC AAG AGC GAT AAT AAT CAC AAG AGC GAT CAT AAT CAC AAG AGC GAT
    150             165             180

AAT AAT CAC AAG AGC GAT CAC AAT CAC AAG AGT GAT AGT AAT CAC AAG
195             210             225             240

AGT GAT AGT AAC CAC AAG AGT GAT AGT AAC CAC AAG AGT GAT AGT AAC
        255             270             285

CAC AAG AGT GAT AAT AAC CAT AAG AGC GAT CAT AAT CAC
        300             315
```

**6.** Séquence nucléotidique de <u>Plasmodium falciparum</u> selon l'une quelconque des revendications 1 à 3, caractérisée en ce qu'elle comprend l'enchainement nucléotidique R51 répondant à la séquence suivante :

```
CAC AAT CAC ATG AGC GAT CAT AAT CAC AAG AGT GAT AAT AAC CAT AAG
1               15              30              45

AGT GAT CAT AAT CAC AAG AGT GAT AGT AAC CAC ATG AGC GAT CAT AAC
        60              75              90

CAC ATG AGC GAT CAT AAC CAC AAG AGC GAT CAT AAT CAC AAG AGC GAT
        105             120             135

CAT AAC CAC AAG AGC GAT AAT AAC CAC AAA AGT GAT AGT AAC CAC AAA
    150             165             180

AGT GA
195
```

**7.** Séquence nucléotidique de <u>Plasmodium falciparum</u> selon l'une quelconque des revendications 1 à 6, caractérisée en ce qu'elle comprend l'enchaînement suivant ou toute partie de cet enchaînement capable dé coder pour un antigène susceptible d'être reconnu par des anticorps contre <u>P falciparum</u> ou d'induire de tels anticorps,

CT

GAG GAA TGT CAA GGG GAA GTT TAT TTA TTT GTT AAA AAG ATA CCT ATA

GAG GTA TGG ATA AGA CAG TAC AAC TTG ATG AAT GAT AAT GAT GGA GAA

TAT TTA TTA GAT GGG GAA AAT TTT ATT ATG GAA GCA GTA GCC TGC GCT

TAT TTA AGC GAG CAT TAT CCT GGA CTC ACA CCT AAA TTG TAC AAG GTA

TTA TAT GAG CCT GAA TGT GCT AAC TGT AAT GAA GAG GAT AAG AAT ATG

AGT GAA GAT AAT CAT AAG AAG GAT AGT AAA CAT AAG GGG GAT AGT AAT

CAC AAA AGT GAT AGT AAT CAC AAA AGT GAT AGT AAT CAC AAA AGT GAT

AGT AAT CAT AAA AGT GAT AGT AAT CAT AAA AGT GGT AGT AAT CAC AAA

AGT GAT TGT AAT CAC AAG AGT GGT AGT AAT CAC AAG AGT GGT AGT AAT

CAC AAG AGT GAT AGT AAT CAT CAA AGT GAT TGT AAT ... ... ... ...

... ... ... ... ... ... ... ... ... GAT CAT AAT CAC AAA AGC GAT

CAT AAC CAC AAG AGC GAT AAT AAC CAC AAG AGT GAT CAT AAT CAC AAA

AGT GAT CAT AAA AAA AAT AAT AAC AAT AAT AAG GAT AAT AAA AAT GAC

GAT AAT GAT GAC AGT GAT GCA AGC GAT GCA GTT GAT GAA GAT ATT GAG

ATA CTT GAG TCT TAT AGT GAT TTG AAT AAA TTT AAT GAG ATG TTA ACA

GAA CAA TTA AAT AAA AAT AAG GAT GGA TAT GTT GTT TTG GTT ACT GAA

TTA TTT GGT GAA GAT CTT TTT CAA TAT ATT AAT AAA AGG AAT GAA AAT

GAA GAT ACG CGT GTA AGA GAT GAA GAT AAA AAA ATA ATT ATG TTT GAA

TTT TTA AAG TTA TTA ATA AAA TTA CAT AAT GCA GGA TTG GTA CAT CTT

GAT ATA TCT CCT GAA AAT ATA TTG ATA GAA AAT AAT GGT GAG TTA CGC

TTA TGT GAT CTA GCT AAA TGT GCT CCA ATG TAT ACA CAC AAT TTA AGA

29

```
CAT ATT AAA GGA AAT GGA AAC GAT TTG TAT TCA TTT CAA TCT TGT CAA

CCT TGT GTT GGA AAA ATC CCA TGT ATT CCA AAA GAG TGT TGG GAT ATT

ATT AGA GAA CAT ATA AAA TTA AAA ATA GAT AAT CCT TTT GAA CAT TTA

TCA ACT ATT ACT GAT CAA GAA GAA AGA AAA AAA TAT TAT TTT GAT GTA

CAT TGT GTA GAT AAA TAT ATG CTT GGT ATA TTT TAT ATA TGG ATA TGG


AAT TTA AAT TAT ATA TGG AAA CGA GCT GAC CCA CCA AAT GAT AGA ACA

TTT AAT AAT TTC TTA AAA TAT AAT TTA AAT ATA AAT GTA TTT CAG TTA

GCC CAA CAA TGG CCA AAA GGT CTC AAA GAT ATA ATT AAC GTA AGA TAT

ATA AGA AAA AAA AAA AAA AAA AAA AAA ATA ATA ATA ATA ATA ATG ATG

TTT TAT ATA GTA TGT GCA TAG TGAATGTTTT TTTTTTATAT TTTAATACAT


GAAATGATAT ATATATATAT ATATATATAT ATATTTTCTC TTTATAGAAA
TTATTAAGCC TAGAAAGTAG AATGAAGACA GACTTAAATG AATTAACTGA
ACATCCATGG TGGATTAATG AAGATTAATT TTAATTTTTT AAGTATTATA
TGAATATTTA TTATTAGTAG ACTTTATATA TAACAATAAA TATTGACACG
TGCAATATTA AAAAAAAAAA AAAAAAAAAA AAAAAAAGA ATTAAAAGG
AATGTTTTAT TGTTTAGGAC TATATGGAAA AATAATAATA TATATATGTT
TCCACATTAA AAA
```

la partie encadrée correspondant à la séquence nucléotidique constituant les "n" répétitions de 18 pb, "n" étant suffisant pour que l'enchaînement formé code pour un antigène susceptible d'être reconnu par des anticorps contre P falciparum ou d'induire de tels anticorps,
le signe (...) représentant des triplets de nucléotides ou codons répétés.

8.  Séquence nucléotidique de P falciparum caractérisée en ce qu'elle répond à l'un des enchaînements I ou II suivants ou à une partie de ces enchaînements codant pour un antigène reconnu par des anticorps contre P falciparum ou susceptible d'induire ou de contribuer à la production in vivo de tels anticorps.

I :

CT

GAG GAA TGT CAA GGG GAA GTT TAT TTA TTT GTT AAA AAG ATA CCT ATA

GAG GTA TGG ATA AGA CAG TAC AAC TTG ATG AAT GAT AAT GAT GGA GAA

TAT TTA TTA GAT GGG GAA AAT TTT ATT ATG GAA GCA GTA GCC TGC GCT

TAT TTA AGC GAG CAT TAT CCT GGA CTC ACA CCT AAA TTG TAC AAG GTA

TTA TAT GAG CCT GAA TGT GCT AAC TGT AAT GAA GAG GAT AAG AAT ATG

AGT GAA GAT AAT

II:                   AAA AAT AAT AAC AAT AAT AAG GAT AAT AAA AAT GAC

GAT AAT GAT GAC AGT GAT GCA AGC GAT GCA GTT GAT GAA GAT ATT GAG

ATA CTT GAG TCT TAT AGT GAT TTG AAT AAA TTT AAT GAG ATG TTA ACA

GAA CAA TTA AAT AAA AAT AAG GAT GGA TAT GTT GTT TTG GTT ACT GAA

TTA TTT GGT GAA GAT CTT TTT CAA TAT ATT AAT AAA AGG AAT GAA AAT

GAA GAT ACG CGT GTA AGA GAT GAA GAT AAA AAA ATA ATT ATG TTT GAA

TTT TTA AAG TTA TTA ATA AAA TTA CAT AAT GCA GGA TTG GTA CAT CTT

GAT ATA TCT CCT GAA AAT ATA TTG ATA GAA AAT AAT GGT GAG TTA CGC

TTA TGT GAT CTA GCT AAA TGT GCT CCA ATG TAT ACA CAC AAT TTA AGA

CAT ATT AAA GGA AAT GGA AAC GAT TTG TAT TCA TTT CAA TCT TGT CAA

CCT TGT GTT GGA AAA ATC CCA TGT ATT CCA AAA GAG TGT TGG GAT ATT

ATT AGA GAA CAT ATA AAA TTA AAA ATA GAT AAT CCT TTT GAA CAT TTA

TCA ACT ATT ACT GAT CAA GAA GAA AGA AAA AAA TAT TAT TTT GAT GTA

CAT TGT GTA GAT AAA TAT ATG CTT GGT ATA TTT TAT ATA TGG ATA TGG

```
AAT TTA AAT TAT ATA TGG AAA CGA GCT GAC CCA CCA AAT GAT AGA ACA

TTT AAT AAT TTC TTA AAA TAT AAT TTA AAT ATA AAT GTA TTT CAG TTA

GCC CAA CAA TGG CCA AAA GGT CTC AAA GAT ATA ATT AAC GTA AGA TAT

ATA AGA AAA AAA AAA AAA AAA AAA AAA ATA ATA ATA ATA ATA ATG ATG

TTT TAT ATA GTA TGT GCA TAG TGAATGTTTT TTTTTTATAT TTTAATACAT


GAAATGATAT ATATATATAT ATATATATAT ATATTTTCTC TTTATAGAAA
TTATTAAGCC TAGAAAGTAG AATGAAGACA GACTTAAATG AATTAACTGA
ACATCCATGG TGGATTAATG AAGATTAATT TTAATTTTTT AAGTATTATA
TGAATATTTA TTATTAGTAG ACTTTATATA TAACAATAAA TATTGACACG
TGCAATATTA AAAAAAAAAA AAAAAAAAAA AAAAAAAGA ATTAAAAAGC
AATGTTTTAT TGTTTAGGAC TATATGGAAA AATAATAATA TATATATGTT
TCCACATTAA AAA
```

9. Antigène de P falciparum susceptible d'induire la formation d'anticorps en particulier d'anticorps protecteurs contre P falciparum, caractérisé en ce qu'il s'agit du produit de traduction d'une séquence selon l'une quelconque des revendications 1 à 8.

10. Antigène de P falciparum caractérisé en ce que :

- il est susceptible d'induire des anticorps et en particulier des anticorps protecteurs contre P falciparum,
- il comprend une séquence d'acides aminés comportant un oligopeptide constitué de n répétitions de peptides identiques ou non, répondant à la formule Asp b His Cys ser, dans laquelle b est un acide aminé quelconque et de préférence Asn, His, Ser ou Cys ou une variante obtenue par substitution d'un ou plusieurs acides aminés dès lors que les propriétés antigéniques et/ou immunogènes de l'antigène formé sont conservées,
- n est suffisant pour que l'oligopeptide formé soit capable d'induire in vivo des anticorps qui reconnaissent l'antigène codé par la séquence nucléotidique de P falciparum répondant à la carte de restriction suivante :

GENE R45

A : Alu I
Ac : Acc I
R : Rsal
* : codon stop
Rp : ≃ 7 répétitions 45 bp

11. Antigène de <u>P falciparum</u> selon la revendication 10 caractérisé en ce qu'il comprend la séquence d'acides aminés suivantes :

```
Glu Glu Cys Gln Gly Glu Val Tyr Leu Phe Val Lys Lys Ile Pro Ile
Glu Val Trp Ile Arg Gln Tyr Asn Leu Met Asn Asp Asn Asp Gly Glu
Tyr Leu Leu Asp Gly Glu Asn Phe Ile Met Glu Ala Val Ala Cys Ala
Tyr Leu Ser Glu His Tyr Pro Gly Leu Thr Pro Lys Leu Tyr Lys Val
Leu Tyr Glu Pro Glu Cys Ala Asn Cys Asn Glu Glu Asp Lys Asn Met
Ser Glu Asp Asn His Lys Lys Asp Ser Lys His Lys Gly Asp Ser Asn
His Lys Ser Asp Ser Asn His Lys Ser Asp Ser Asn His Lys Ser Asp
Ser Asn His Lys Ser Asp Ser Asn His Lys Ser Gly Ser Asn His Lys
Ser Asp Cys Asn His Lys Ser Gly Ser Asn His Lys Ser Gly Ser Asn
His Lys Ser Asp Ser Asn His Gln Ser Asp Cys Asn ... ... ... ...

... ... ... ... ... ... ... ... ... Asp His Asn His Lys Ser Asp
His Asn His Lys Ser Asp Asn Asn His Lys Ser Asp His Asn His Lys
Ser Asp His Lys Lys Asn Asn Asn Asn Asn Lys Asp Asn Lys Asn Asp
Asp Asn Asp Asp Ser Asp Ala Ser Asp Ala Val Asp Glu Asp Ile Glu
Ile Leu Glu Ser Tyr Ser Asp Leu Asn Lys Phe Asn Glu Met Leu Thr
Glu Gln Leu Asn Lys Asn Lys Asp Gly Tyr Val Val Leu Val Thr Glu
Leu Phe Gly Glu Asp Leu Phe Gln Tyr Ile Asn Lys Arg Asn Glu Asn
Glu Asp Thr Arg Val Arg Asp Glu Asp Lys Lys Ile Ile Met Phe Glu
Phe Leu Lys Leu Leu Ile Lys Leu His Asn Ala Gly Leu Val His Leu
Asp Ile Ser Pro Glu Asn Ile Leu Ile Glu Asn Asn Gly Glu Leu Arg
Leu Cys Asp Leu Ala Lys Cys Ala Pro Met Tyr Thr His Asn Leu Arg
His Ile Lys Gly Asn Gly Asn Asp Leu Tyr Ser Phe Gln Ser Cys Gln
Pro Cys Val Gly Lys Ile Pro Cys Ile Pro Lys Glu Cys Trp Asp Ile
```

```
Ile Arg Glu His Ile Lys Leu Lys Ile Asp Asn Pro Phe Glu His Leu

Ser Thr Ile Thr Asp Gln Glu Glu Arg Lys Lys Tyr Tyr Phe Asp Val

His Cys Val Asp Lys Tyr Met Leu Gly Ile Phe Tyr Ile Trp Ile Trp

Asn Leu Asn Tyr Ile Trp Lys Arg Ala Asp Pro Pro Asn Asp Arg Thr

Phe Asn Asn Phe Leu Lys Tyr Asn Leu Asn Ile Asn Val Phe Gln Leu

Ala Gln Gln Trp Pro Lys Gly Leu Lys Asp Ile Ile Asn Val Arg Tyr

Ile Arg Lys Lys Lys Lys Lys Lys Lys Ile Ile Ile Ile Ile Met Met

Phe Tyr Ile Val Cys Ala,
```

la partie encadrée correspondant à la séquence peptidique constituant les "n" répétitions de 6 acides aminés, "n" étant défini tel que dans la revendication 10,
le signe (...) représentant un acide aminé de la séquence répétée.

12. Antigène de P falciparum selon la revendication 10 caractérisé en ce qu'il comprend l'une des séquences d'acides aminés R23, R45 ou R 51 suivantes :

**R23**

LysSerAspSerAsnHisLysSerAspHisAsnHisLysSerAspSerAsn

HisMetSerAspHisAsnHisMetSerAspHisAsnHisLysSerAspHisAsnHisLys

SerAspAsnAsnHisLysSerAspSerAsnHisLysSerAspSerAsnHisLysSerAsp

SerAsnHisLysSerAspHisAsn

**R51**

HisAsnHisMetSerAspHisAsnHisLysSerAspAsnAsnHisLysSerAspHis

AsnHisLysSerAspSerAsnHisMetSerAspHisAsnHisMetSerAspHisAsnHis

LysSerAspHisAsnHisLysSerAspHisAsnHisLysSerAspAsnAsnHisLysSer

AspSerAsnHisLysSer

**R45**

```
HisLysSerAspAsnAsnHisLysSerAspSerAsnHisLysSerAspSerAsn

HisLysSerAspSerAsnHisLysSerAspHisAsnHisLysSerAspHisLysHisMet

SerAspAsnAsnHisLysSerAspAsnAsnHisLysSerAspAsnAsnHisLysSerAsp

HisAsnHisLysSerAspAsnAsnHisLysSerAspHisAsnHisLysSerAspSerAsn

HisLysSerAspSerAsnHisLysSerAspSerAsnHisLysSerAspSerAsnHisLys

SerAspAsnAsnHisLysSerAspHisAsnHis
```

13. Antigène de P falciparum selon l'une quelconque des revendications 9 à 12, caractérisé en ce qu'il est susceptible d'induire in vivo la production d'anticorps protecteurs cytophiles, notamment des anticorps $IgG_1$, $IgG_3$.

14. Vecteur recombinant de clonage et/ou d'expression, caractérisé en ce qu'il comprend une séquence nucléotidique selon l'une quelconque des revendications 1 à 8, le cas échéant sous le contrôle d'un promoteur et des éléments de régulation pour l'expression dans un hôte cellulaire déterminé.

15. Vecteur recombinant selon la revendication 14 caractérisé en ce que le vecteur utilisé est pMS, λgt11, ou pGEX.

16. Polypeptide recombinant caractérisé en qu'il comprend une séquence antigénique selon l'une quelconque des revendications 9 à 13, en association avec un polypeptide, notamment un polypeptide caractéristique d'un stade sanguin ou hépatique de l'infection de P falciparum, par exemple l'antigène 96TR ou encore un polypeptide ou une protéine porteuse notamment la β-galactosidase, l'anatoxine tétanique, l'ovalbumine, la sérum albumine, la glutathione transférase ou encore une molécule porteuse synthétique.

17. Hôte cellulaire recombinant, caractérisé en ce qu'il est transformé par un vecteur selon l'une quelconque des revendications 14 ou 15, dans des conditions permettant l'expression de la séquence de P falciparum selon l'une quelconque des revendications 1 à 8, et en ce qu'il s'agit notamment d'une bactérie telle que E.coli, d'une levure, d'une cellule d'insecte ou d'une cellule de mammifère telle que CHO.

18. Hôte cellulaire selon la revendication 17, caractérisé en ce qu'il s'agit de la souche SB275 déposée à la CMCM sous le n°I-986 le 27 Juillet 1990.

19. Hôte cellulaire selon la revendication 18, caractérisé en ce qu'il s'agit de la souche SB363 déposée à la CNCM sous le n°I-987 le 27 Juillet 1990.

20. Composition vaccinante, caractérisée en ce qu'elle comprend à titre de principe actif un antigène selon l'une quelconque des revendications 9 à 14.

21. Composition vaccinante selon la revendication 20, caractérisée en ce qu'elle contient en outre un autre antigène des stades sanguin ou hépatique de P falciparum.

22. Antigène caractérisé en ce qu'il est reconnu par des anticorps formés contre la séquence selon la revendication 11, en ce qu'il a un poids moléculaire d'environ 160kDa et en ce qu'il est présent à un stade précoce de l'infection par P falciparum.

23. Séquence peptidique caractérisée en ce qu'elle est reconnue par des anticorps dirigés contre P falciparum, et le cas échéant en ce qu'elle est capable d'induire la formation d'anticorps contre P falciparum, ou de contribuer à la formation de tels anticorps, et en particulier en ce qu'il s'agit de la séquence suivante HKSDNNHKSDSNHKSDSN.

24. Fragments d'ADN ou d'ARN, utilisables comme amorces, constitués d'environ 8 à 25 nucléotides identiques à une séquence de même longueur d'une séquence selon l'une quelconque des revendications 1 à 8, ou fragments d'ADN ou d'ARN capables d'hybrider avec une séquence selon l'une quelconque des revendications 1 à 8 dans un milieu répondant aux conditions suivantes 6 x SSC, 2,5 % lait, 100 µg/ml DNA hareng 65°C avec des étapes de lavage dans un milieu contenant 6 x SSC et 0,5 SSC.

**Patentansprüche**

1. Nukleotidsequenz von P falciparum, dadurch gekennzeichnet, daß:

- sie aus einer Nukleotidkette besteht, die n aufeinanderfolgende Wiederholungen von identischen oder nicht-identischen Nukleotidfragmenten aus 18 Basenpaaren (bp) umfaßt, wobei die Nukleotidfragmente codieren

- für identische oder nicht-identische Peptide aus 6 Aminosäuren entsprechend der folgenden Grundeinheit

$$\text{Asp b Asn His Lys Ser}$$

wobei b eine beliebige Aminosäure ist, oder
- für eine variante dieser Grundeinheit, die durch Substitution einer oder mehrerer Aminosäuren erhalten wurde, ohne daß die Antigen- und/oder Immunogen-eigenschaften des gebildeten Oligopeptids verändert wurden,

- ausgenommen, daß die gebildete Nukleotidkette für ein Oligopeptid codiert, das in vivo Antikörper induzieren kann, die ein Antigen erkennen können, das durch die Nukleotidsequenz von P falciparum entsprechend der folgenden Restriktionskarte codiert wird:

2. Nukleotidsequenz nach Anspruch 1, dadurch gekennzeichnet, daß die Grundeinheit modifiziert ist und einem der folgenden Peptide entspricht:

```
Asp Ser Asn His Met Ser
Asp Ser Asn His Met Ser
    Ser Asn His Met Ser
Glu Ser Asn His Lys Ser
Asp Ser Asn His Glu Ser
```

3.  Nukleotidsequenz nach Anspruch 1 oder 2, dadurch gekennzeichnet, daß b vorzusgweise Asn, His, Ser, Cys ist.

4.  Nukleotidsequenz von Plasmodium falciparum nach irgendeinem der Ansprüche 1 bis 3, dadurch gekennzeichnet, daß sie eine Nukleotidkette R23 entsprechend der folgenden Sequenz umfaßt:

```
                                                              AC
AAA AGT GAT AGT AAC CAC AAG AGC GAT CAT AAT CAC AAG AGT GAT AGT

AAC CAC ATG AGC GAT CAT AAC CAC ATG AGC GAT CAT AAC CAC AAG AGC

GAT CAT AAC CAC AAG AGC GAT AAT AAC CAC AAA AGT GAT AGT AAC CAC

AAA AGT GAT AGT AAC CAC AAA AGT GAT AGT AAC CAC AAG AGC GAT CAT

AAT C
```

5.  Nukleotidsequenz von Plasmodium falciparum nach einem der Ansprüche 1 bis 3, dadurch gekennzeichnet, daß sie eine Nukleotidkette R45 entsprechend der folgenden Sequenz umfaßt:

```
CAC AAG AGC GAT AAT AAC CAC AAA AGT GAT AGT AAC CAC AAA AGT GAT
1               15                30              45

AGT AAC CAC AAA AGT GAT AGT AAC CAC AAG AGC GAT CAT AAT CAC AAG
            60              75                  90

AGC GAT CAT AAG CAC ATG AGC GAT AAT AAC CAC AAG AGC GAT AAT AAT
        105             120                 135

CAC AAG AGC GAT AAT AAT CAC AAG AGC GAT CAT AAT CAC AAG AGC GAT
    150                 165                 180

AAT AAT CAC AAG AGC GAT CAC AAT CAC AAG AGT GAT AGT AAT CAC AAG
195                 210                 225                 240

AGT GAT AGT AAC CAC AAG AGT GAT AGT AAC CAC AAG AGT GAT AGT AAC
                255                 270                 285

CAC AAG AGT GAT AAT AAC CAT AAG AGC GAT CAT AAT CAC
            300             315
```

6.  Nukleotidsequenz von Plasmodium falciparum nach einem der Ansprüche 1 bis 3, dadurch gekennzeichnet, daß sie eine Nukleotidkette R51 entsprechend der folgenden Sequenz umfaßt:

```
CAC AAT CAC ATG AGC GAT CAT AAT CAC AAG AGT GAT AAT AAC CAT AAG
1                15                30                45

AGT GAT CAT AAT CAC AAG AGT GAT AGT AAC CAC ATG AGC GAT CAT AAC
            60              75                90

CAC ATG AGC GAT CAT AAC CAC AAC AGC GAT CAT AAT CAC AAG AGC GAT
        105             120                135

CAT AAC CAC AAG AGC GAT AAT AAC CAC AAA AGT GAT AGT AAC CAC AAA
    150             165             180

AGT GA
195
```

7. Nukleotidsequenz von Plasmodium falciparum nach einem der Ansprüche 1 bis 6, dadurch gekennzeichnet, daß sie die folgende Kette umfaßt oder einen zusammenhängenden Teil dieser Kette, der für ein Antigen codiert, das durch ein Antikörper gegen P falciparum erkannt oder durch diesen Antikörper induziert werden kann,

CT

GAG GAA TGT CAA GGC GAA GTT TAT TTA TTT GTT AAA AAG ATA CCT ATA·

GAG GTA TGG ATA AGA CAG TAC AAC TTG ATG AAT GAT AAT GAT GGA GAA

TAT TTA TTA GAT GGC GAA AAT TTT ATT ATG GAA GCA GTA GCC TGC GCT

TAT TTA AGC GAG CAT TAT CCT GGA CTC ACA CCT AAA TTG TAC AAG GTA

TTA TAT GAG CCT GAA TGT GCT AAC TGT AAT GAA GAG GAT AAG AAT ATG

AGT GAA GAT AAT ┌CAT AAG AAG GAT AGT AAA¸ CAT AAG GGG GAT AGT AAT

CAC AAA AGT GAT AGT AAT CAC AAA AGT GAT AGT AAT CAC AAA AGT GAT

AGT AAT CAT AAA AGT GAT AGT AAT CAT AAA AGT GGT AGT AAT CAC AAA

AGT GAT TGT AAT CAC AAG AGT GGT AGT AAT CAC AAG AGT GGT AGT AAT

CAC AAG AGT GAT AGT AAT CAT CAA AGT GAT TGT AAT ... ... ... ...

... ... ... ... ... ... ... ... ... GAT CAT AAT CAC AAA AGC GAT

CAT AAC CAC AAG AGC GAT AAT AAC CAC AAG AGT GAT CAT AAT CAC AAA

AGT GAT CAT AAA┘AAA AAT AAT AAC AAT AAT AAG GAT AAT AAA AAT GAC

GAT AAT GAT GAC AGT GAT GCA AGC GAT GCA GTT GAT GAA GAT ATT GAG

ATA CTT GAG TCT TAT AGT GAT TTG AAT AAA TTT AAT GAG ATG TTA ACA

GAA CAA TTA AAT AAA AAT AAG GAT GGA TAT GTT GTT TTC GTT ACT GAA

TTA TTT GGT GAA GAT CTT TTT CAA TAT ATT AAT AAA AGG AAT GAA AAT

GAA GAT ACG CGT GTA AGA GAT GAA GAT AAA AAA ATA ATT ATG TTT GAA

TTT TTA AAG TTA TTA ATA AAA TTA CAT AAT GCA GGA TTC GTA CAT CTT

GAT ATA TCT CCT GAA AAT ATA TTG ATA GAA AAT AAT GCT GAC TTA CGC

TTA TGT GAT CTA GCT AAA TGT GCT CCA ATG TAT ACA CAT AAT TTA AGA

39

```
CAT ATT AAA CGA AAT GGA AAC GAT TTG TAT TCA TTT CAA TCT TGT CAA
CCT TGT GTT GGA AAA ATC CCA TGT ATT CCA AAA GAG TGT TGG GAT ATT
ATT AGA GAA CAT ATA AAA TTA AAA ATA GAT AAT CCT TTT GAA CAT TTA
TCA ACT ATT ACT GAT CAA GAA GAA AGA AAA AAA TAT TAT TTT GAT GTA
CAT TGT GTA GAT AAA TAT ATG CTT GGT ATA TTT TAT ATA TGG ATA TGG


AAT TTA AAT TAT ATA TGG AAA CGA GCT GAC CCA CCA AAT GAT AGA ACA
TTT AAT AAT TTC TTA AAA TAT AAT TTA AAT ATA AAT GTA TTT CAG TTA
GCC CAA CAA TGG CCA AAA GGT CTC AAA GAT ATA ATT AAC GTA AGA TAT
ATA AGA AAA AAA AAA AAA AAA AAA AAA ATA ATA ATA ATA ATA ATG ATG
TTT TAT ATA GTA TGT GCA TAG TGAATGTTTT TTTTTTATAT TTTAATACAT


GAAATCATAT ATATATATAT ATATATATAT ATATTTTCTC TTTATAGAAA
TTATTAAGCC TAGAAAGTAG AATGAAGACA GACTTAAATG AATTAACTGA
ACATCCATGG TGGATTAATG AAGATTAATT TTAATTTTTT AAGTATTATA
TGAATATTTA TTATTAGTAG ACTTTATATA TAACAATAAA TATTGACACG
TGCAATATTA AAAAAAAAAA AAAAAAAAAA AAAAAAAAGA ATTAAAAAGG
AATGTTTTAT TGTTTAGGAC TATATGGAAA AATAATAATA TATATATGTT
TCCACATTAA AAA
```

der eingerahmte Bereich entspricht einer Nukleotidsequenz aus "n" Wiederholungen von 18 pb, wobei "n" ausreichend ist, daß die gebildete Kette für ein Antigen codiert, das durch Antikörper gegen P falciparum erkannt oder durch diese Antikörper induziert werden kann,
die Zeichen (...) stehen für Nukleotidtriplets oder wiederholte Codons.

8.  Nukleotidsequenz von P falciparum, dadurch gekennzeichnet, daß sie einer der folgenden Ketten I oder II entspricht oder einem Teil dieser Ketten, die für ein Antigen codieren, das durch Antikörper gegen P falciparum erkannt oder induziert werden kann oder an der in vivo Erzeugung dieser Antikörper teilnimmt:

1 :

CT

GAG GAA TGT CAA GGG GAA GTT TAT TTA TTT GTT AAA AAG ATA CCT ATA

GAG CTA TGG ATA AGA CAG TAC AAC TTG ATG AAT GAT AAT GAT GGA GAA

TAT TTA TTA GAT GGG GAA AAT TTT ATT ATG GAA GCA GTA GCC TGC GCT

TAT TTA AGC GAG CAT TAT CCT GGA CTC ACA CCT AAA TTG TAC AAG CTA

TTA TAT GAG CCT GAA TGT GCT AAC TGT AAT GAA GAG GAT AAG AAT ATG

AGT CAA GAT AAT

II:                          AAA AAT AAT AAC AAT AAT AAC GAT AAT AAA AAT GAC

GAT AAT GAT GAC AGT GAT GCA AGC GAT GCA GTT GAT GAA GAT ATT GAG

ATA CTT GAG TCT TAT AGT GAT TTG AAT AAA TTT AAT GAG ATG TTA ACA

GAA CAA TTA AAT AAA AAT AAG GAT GGA TAT GTT GTT TTG GTT ACT GAA

TTA TTT CGT GAA GAT CTT TTT CAA TAT ATT AAT AAA ACG AAT GAA AAT

GAA GAT ACG CGT GTA AGA GAT GAA GAT AAA AAA ATA ATT ATG TTT GAA

TTT TTA AAG TTA TTA ATA AAA TTA CAT AAT GCA GGA TTG GTA CAT CTT

GAT ATA TCT CCT GAA AAT ATA TTG ATA GAA AAT AAT GGT GAG TTA CGC

TTA TGT GAT CTA GCT AAA TGT GCT CCA ATG TAT ACA CAC AAT TTA AGA

CAT ATT AAA GGA AAT GGA AAC GAT TTG TAT TCA TTT CAA TCT TGT CAA

CCT TGT GTT GGA AAA ATC CCA TGT ATT CCA AAA GAG TGT TCG GAT ATT

ATT AGA GAA CAT ATA AAA TTA AAA ATA GAT AAT CCT TTT GAA CAT TTA

TCA ACT ATT ACT GAT CAA GAA GAA ACA AAA AAA TAT TAT TTT GAT GTA

CAT TGT GTA GAT AAA TAT ATG CTT GCT ATA TTT TAT ATA TGG ATA TGG

EP 0 542 845 B1

```
AAT TTA AAT TAT ATA TGG AAA CGA GCT GAC CCA CCA AAT GAT AGA ACA

TTT AAT AAT TTC TTA AAA TAT AAT TTA AAT ATA AAT GTA TTT CAG TTA

GCC CAA CAA TGG CCA AAA GGT CTC AAA GAT ATA ATT AAC GTA AGA TAT

ATA AGA AAA AAA AAA AAA AAA AAA AAA ATA ATA ATA ATA ATA ATC ATG

TTT TAT ATA GTA TGT GCA TAG TGAATGTTTT TTTTTTATAT TTTAATACAT


GAAATGATAT ATATATATAT ATATATATAT ATATTTTCTC TTTATAGAAA
TTATTAAGCC TAGAAAGTAG AATGAAGACA GACTTAAATG AATTAACTGA
ACATCCATGG TGGATTAATG AAGATTAATT TTAATTTTTT AAGTATTATA
TGAATATTTA TTATTAGTAG ACTTTATATA TAACAATAAA TATTGACACG
TGCAATATTA AAAAAAAAAA AAAAAAAAAA AAAAAAAGA ATTAAAAGG
AATGTTTTAT TGTTTAGGAC TATATGGAAA AATAATAATA TATATATGTT
TCCACATTAA AAA
```

9. Antigen von P falciparum, das die Bildung von Antikörpern induzieren kann, insbesondere von gegen P falciparum schützenden Antikörpern, dadurch gekennzeichnet, daß es sich um ein übersetzungsprodukt aus einer Sequenz nach einem der Ansprüche 1 bis 8 handelt.

10. Antigen von P falciparum, dadurch gekennzeichnet, daß:

- es ist in der Lage ist, Antikörper zu induzieren und insbesondere schützende Antikörper gegen P falciparum,

- es eine Aminosäuresequenz umfaßt, enthaltend ein oligopeptid, das aus n Wiederholungen von identischen oder nicht-identischen Peptiden, entsprechend der Formel Asp b His Cys Ser besteht, wobei b eine beliebige Aminosäure ist, vorzugsweise Asn, His, Ser oder Cys oder eine durch Substitution mit einer oder mehreren Aminosäuren erhaltene Variante, wobei die Antigen- und/oder Immunogeneigenschaften des gebildeten Antigens beibehalten werden,

- wobei ausgenommen wird, daß das gebildete Oligopeptid in vivo Antikörper induzieren kann, die ein Antigen erkennen, das durch die Nukleotidsequenz von P falciparum entsprechend der folgenden Restriktionskarte codiert wird:

GEN R45

≈80 Wiederholungen 18bp

Ddel

A : Alu I
Ac : Acc I
R : Rsal
* : codon stop
Rp : ≈7 Wiederholungen 45bp

11. Antigen von P falciparum nach Anspruch 10, dadurch gekennzeichnet, daß es die folgende Aminosäurensequenz umfaßt:

EP 0 542 845 B1

Glu Glu Cys Gln Gly Glu Val Tyr Leu Phe Val Lys Lys Ile Pro Ile

Glu Val Trp Ile Arg Gln Tyr Asn Leu Met Asn Asp Asn Asp Gly Glu

Tyr Leu Leu Asp Gly Glu Asn Phe Ile Met Glu Ala Val Ala Cys Ala

Tyr Leu Ser Glu His Tyr Pro Gly Leu Thr Pro Lys Leu Tyr Lys Val

Leu Tyr Glu Pro Glu Cys Ala Asn Cys Asn Glu Glu Asp Lys Asn Met

Ser Glu Asp Asn His Lys Lys Asp Ser Lys His Lys Gly Asp Ser Asn

His Lys Ser Asp Ser Asn His Lys Ser Asp Ser Asn His Lys Ser Asp

Ser Asn His Lys Ser Asp Ser Asn His Lys Ser Gly Ser Asn His Lys

Ser Asp Cys Asn His Lys Ser Gly Ser Asn His Lys Ser Gly Ser Asn

His Lys Ser Asp Ser Asn His Gln Ser Asp Cys Asn ... ... ... ...

... ... ... ... ... ... ... ... ... Asp His Asn His Lys Ser Asp

His Asn His Lys Ser Asp Asn Asn His Lys Ser Asp His Asn His Lys

Ser Asp His Lys Lys Asn Asn Asn Asn Asn Lys Asp Asn Lys Asn Asp

Asp Asn Asp Asp Ser Asp Ala Ser Asp Ala Val Asp Glu Asp Ile Glu

Ile Leu Glu Ser Tyr Ser Asp Leu Asn Lys Phe Asn Glu Met Leu Thr

Glu Gln Leu Asn Lys Asn Lys Asp Gly Tyr Val Val Leu Val Thr Glu

Leu Phe Gly Glu Asp Leu Phe Gln Tyr Ile Asn Lys Arg Asn Glu Asn

Glu Asp Thr Arg Val Arg Asp Glu Asp Lys Lys Ile Ile Met Phe Glu

Phe Leu Lys Leu Leu Ile Lys Leu His Asn Ala Gly Leu Val His Leu

Asp Ile Ser Pro Glu Asn Ile Leu Ile Glu Asn Asn Gly Glu Leu Arg

Leu Cys Asp Leu Ala Lys Cys Ala Pro Met Tyr Thr His Asn Leu Arg

His Ile Lys Gly Asn Gly Asn Asp Leu Tyr Ser Phe Gln Ser Cys Gln

Pro Cys Val Gly Lys Ile Pro Cys Ile Pro Lys Glu Cys Trp Asp Ile

44

```
Ile Arg Glu His Ile Lys Leu Lys Ile Asp Asn Pro Phe Glu His Leu
Ser Thr Ile Thr Asp Gln Glu Glu Arg Lys Lys Tyr Tyr Phe Asp Val
His Cys Val Asp Lys Tyr Met Leu Gly Ile Phe Tyr Ile Trp Ile Trp
Asn Leu Asn Tyr Ile Trp Lys Arg Ala Asp Pro Pro Asn Asp Arg Thr
Phe Asn Asn Phe Leu Lys Tyr Asn Leu Asn Ile Asn Val Phe Gln Leu
Ala Gln Gln Trp Pro Lys Gly Leu Lys Asp Ile Ile Asn Val Arg Tyr
Ile Arg Lys Lys Lys Lys Lys Lys Lys Ile Ile Ile Ile Ile Met Met
Phe Tyr Ile Val Cys Ala,
```

der eingerahmte Bereich entspricht einer Peptidsequenz, die "n" Wiederholungen von 6 Aminosäuren ausmacht, wobei "n" so wie in Anspruch 10 definiert ist,

die Zeichen (...) stehen für eine Aminosäure einer sich wiederholenden sequenz.

12. Antigen von P falciparum nach Anspruch 10, dadurch gekennzeichnet, daß es eine der folgenden Aminosäure-sequenzen R23, R45 oder R51 umfaßt:

**R23**

LysSerAspSerAsnHisLysSerAspHisAsnHisLysSerAspSerAsn

HisMetSerAspHisAsnHisMetSerAspHisAsnHisLysSerAspHisAsnHisLys

SerAspAsnAsnHisLysSerAspSerAsnHisLysSerAspSerAsnHisLysSerAsp

SerAsnHisLysSerAspHisAsn

**R51**

HisAsnHisMetSerAspHisAsnHisLysSerAspAsnAsnHisLysSerAspHis

AsnHisLysSerAspSerAsnHisMetSerAspHisAsnHisMetSerAspHisAsnHis

LysSerAspHisAsnHisLysSerAspHisAsnHisLysSerAspAsnAsnHisLysSer

AspSerAsnHisLysSer

**R45**

HisLysSerAspAsnAsnHisLysSerAspSerAsnHisLysSerAspSerAsn

HisLysSerAspSerAsnHisLysSerAspHisAsnHisLysSerAspHisLysHisMet

SerAspAsnAsnHisLysSerAspAsnAsnHisLysSerAspAsnAsnHisLysSerAsp

HisAsnHisLysSerAspAsnAsnHisLysSerAspHisAsnHisLysSerAspSerAsn

HisLysSerAspSerAsnHisLysSerAspSerAsnHisLysSerAspSerAsnHisLys

SerAspAsnAsnHisLysSerAspHisAsnHis

13. Antigen von P falciparum nach einem der Ansprüche 9 bis 12, dadurch gekennzeichnet, daß es in vivo die Erzeugung von schützenden cytophilen Antikörpern induziert, insbesondere die Antikörper $IgG_1$, $IgG_3$.

14. Recombinanter Vektor für die Klonierung und/oder Expression, dadurch gekennzeichnet, daß er eine Nukleotidsequenz nach irgendeinem der Ansprüche 1 bis 8 umfaßt, ggf. unter der Kontrolle eines Promotors und von Regulationselementen für die Expression in vorbestimmten höheren Zellen.

15. Recombinanter Vektor nach Anspruch 14, dadurch gekennzeichnet, daß der eingesetzte Vektor pMS, λgt11 oder pGEX ist.

**16.** Recombinantes Polypeptid, dadurch gekennzeichnet, daß es eine Antigensequenz nach einem der Ansprüche 9 bis 13 umfaßt, in Assoziation mit einem Polypeptid, insbesondere ein Polypeptid, das durch einen sanguinen oder hepatischen Infektionsstatus durch P falciparum gekennzeichnet ist, beispielsweise das Antigen 96TR und darüberhinaus ein Polypeptid oder ein Trägerprotein, insbesondere β-Galactosidase, Tetanusanatoxin, Eiweiß, Serumeiweiß, Glutathiontransferase oder darüberhinaus ein synthetisches Trägermolekül.

**17.** Höhere recombinante Zelle, dadurch gekennzeichnet, daß sie durch einen Vektor nach einem der Ansprüche 14 oder 15 transformiert ist unter Bedingungen, die die Expression der Sequenz von P falciparum nach einem der Ansprüche 1 bis 8 erlauben, und wobei es sich insbesondere um Bakterien, wie E. coli, um einen Pilz, um eine Insektenzelle oder um eine Mammiferezelle, wie CHO, handelt.

**18.** Höhere Zelle nach Anspruch 17, dadurch gekennzeichnet, daß es sich um den Stamm SB275 handelt, der bei der CMCM unter der Nummer I-986 am 27. Juli 1990 hinterlegt wurde.

**19.** Höhere Zelle nach Anspruch 18, dadurch gekennzeichnet, daß es sich um den Stamm SB363 handelt, der bei der CMCM unter der Nummer I-987 am 27. Juli 1990 hinterlegt wurde.

**20.** Impfzusammensetzung, dadurch gekennzeichnet, daß sie als Wirkstoff ein Antigen nach einem der Ansprüche 9 bis 14 umfaßt.

**21.** Impfzusammensetzung nach Anspruch 20, dadurch gekennzeichnet, daß sie u. a. ein anderes Antigen des sanguinen oder hepatischen Stadiums von P falciparum enthält.

**22.** Antigen, dadurch gekennzeichnet, daß es durch Antikörper erkannt wird, die gegen eine Sequenz nach Anspruch 11 gebildet wird, daß es ein Molekulargewicht von etwa 160kDa hat, und daß es bei einem Frühstadium einer Infektion durch P falciparum vorhanden ist.

**23.** Peptidsequenz, dadurch gekennzeichnet, daß sie durch gegen P falciparum gerichtete Antikörper erkannt werden kann, und ggf. dadurch, daß sie die Bildung von Antikörpern gegen P falciparum induzieren kann oder bei der Bildung dieser Antikörper teilnimmt, und insbesondere, daß es sich um die folgende Sequenz handelt HKSDNNHKSDSNHKSDSN.

**24.** Fragmente aus ADN oder ARN, die als Starter verwendet werden können, bestehend aus etwa 8 bis 25 Nukleotiden, die einer gleichlangen Sequenz einer Sequenz nach einem der Ansprüche 1 bis 8 entsprechen, oder Fragmente von ADN oder ARN, die mit einer Sequenz nach einem der Ansprüche 1 bis 8 hybridisieren können in einem Milieu entsprechend den folgenden Bedingungen: 6 x SSC, 2,5 % Milch, 100 µg/ml Hering-DNA, 65°C, mit Waschschritten in einem Milieu aus 6 x SSC und 0,5 SSC.

**Claims**

**1.** Nucleotide sequence of *P. falciparum,* characterized in that:

- it comprises a sequence of nucleotides containing n successive repetitions of identical or non-identical nucleotide fragments of 18 base pairs (bp), the said nucleotide fragments coding

  • for identical or non-identical peptides, of 6 amino acids corresponding to the following base unit: Asp b Asn His Lys Ser in which b is any amino acid, or
  • for a variant of this base unit obtained by substitution of one or more amino acids, provided that the antigenic and/or immunogenic properties of the oligopeptide formed are not modified,

- n is sufficient for the nucleotide sequence formed to code for an oligopeptide capable of inducing, *in vivo,* antibodies which recognize the antigen coded by the nucleotide sequence of *P. falciparum* corresponding to the restriction map given below:

GENE R45

A : Alu I
Ac : Acc I
R : Rsa I
✻ : stop codon
Rp : 7 repetitions 46 bp

2. Nucleotide sequence according to Claim 1, characterized in that the base unit is modified and corresponds to a peptide chosen from:

Asp Ser Asn His Met Ser

Asp Ser Asn His Met Ser

Ser Asn His Met Ser

Glu Ser Asn His Lys Ser

Asp Ser Asn His Glu Ser

3. Nucleotide sequence according to Claim 1 or 2, characterized in that b is preferably Asn, His, Ser or Cys.

4. Nucleotide sequence of *Plasmodium falciparum* according to any one of Claims 1 to 3, characterized in that it comprises the nucleotide sequence R23 corresponding to the following sequence:

AC

AAA AGT GAT AGT AAC CAC AAG AGC GAT CAT AAT CAC AAG AGT GAT AGT

AAC CAC ATG AGC GAT CAT AAC CAC ATG AGC GAT CAT AAC CAC AAG AGC

GAT CAT AAC CAC AAG AGC GAT AAT AAC CAC AAA AGT GAT AGT AAC CAC

AAA AGT GAT AGT AAC CAC AAA AGT GAT AGT AAC CAC AAG AGC GAT CAT

AAT C

5. Nucleotide sequence of *Plasmodium falciparum* according to any one of Claims 1 to 3, characterized in that it comprises the nucleotide sequence R45 corresponding to the following sequence:

```
CAC AAG AGC GAT AAT AAC CAC AAA AGT GAT AGT AAC CAC AAA AGT GAT
1                    15                  30                  45

AGT AAC CAC AAA AGT GAT AGT AAC CAC AAG AGC GAT CAT AAT CAC AAC
                60                  75                  90

AGC GAT CAT AAG CAC ATG AGC GAT AAT AAC CAC AAG AGC GAT AAT AAT
            105                 120                 135

CAC AAG AGC GAT AAT AAT CAC AAG AGC GAT CAT AAT CAC AAG AGC GAT
        150                 165                 180

AAT AAT CAC AAG AGC GAT CAC AAT CAC AAG AGT GAT AGT AAT CAC AAG
195                 210                 225                 240

AGT GAT AGT AAC CAC AAG AGT GAT AGT AAC CAC AAG AGT GAT AGT AAC
                255                 270                 285

CAC AAG AGT GAT AAT AAC CAT AAG AGC GAT CAT AAT CAC
            300                 315
```

6. Nucleotide sequence of *Plasmodium falciparum* according to any one of Claims 1 to 3, characterized in that it comprises the nucleotide sequence R51 corresponding to the following sequence:

```
CAC AAT CAC ATG AGC GAT CAT AAT CAC AAG AGT GAT AAT AAC CAT AAG
1                    15                  30                  45

AGT GAT CAT AAT CAC AAC AGT GAT AGT AAC CAC ATC AGC GAT CAT AAC
                60                  75                  90

CAC ATC AGC GAT CAT AAC CAC AAG AGC GAT CAT AAT CAC AAG AGC GAT
            105                 120                 135

CAT AAC CAC AAG AGC GAT AAT AAC CAC AAA AGT GAT AGT AAC CAC AAA
        150                 165                 180

AGT GA
175
```

7. Nucleotide sequence of *Plasmodium falciparum* according to any one of Claims 1 to 6, characterized in that it comprises the following sequence or any part of this sequence capable of coding for an antigen liable to be recognized by antibodies raised against *P. falciparum* or of inducing such antibodies,

CT

GAG GAA TGT CAA GGG GAA GTT TAT TTA TTT GTT AAA AAG ATA CCT ATA

GAG GTA TGG ATA AGA CAG TAC AAC TTG ATG AAT GAT AAT GAT GGA GAA

TAT TTA TTA GAT GGG GAA AAT TTT ATT ATG GAA GCA GTA CCC TGC GCT

TAT TTA AGC GAG CAT TAT CCT GGA CTC ACA CCT AAA TTC TAC AAG GTA

TTA TAT GAG CCT GAA TGT GCT AAC TGT AAT GAA GAG GAT AAG AAT ATG

AGT GAA GAT AAT CAT AAG AAG GAT AGT AAA CAT AAG GGG GAT AGT AAT

CAC AAA AGT GAT AGT AAT CAC AAA AGT GAT AGT AAT CAC AAA AGT GAT

AGT AAT CAT AAA AGT GAT AGT AAT CAT AAA AGT GGT AGT AAT CAC AAA

AGT GAT TGT AAT CAC AAG AGT GGT AGT AAT CAC AAG AGT GGT AGT AAT

CAC AAG AGT GAT AGT AAT CAT CAA AGT GAT TGT AAT ... ... ... ...

... ... ... ... ... ... ... ... ... GAT CAT AAT CAC AAA AGC GAT

CAT AAC CAC AAG AGC GAT AAT AAC CAC AAG AGT GAT CAT AAT CAC AAA

AGT GAT CAT AAA AAA AAT AAT AAC AAT AAT AAG GAT AAT AAA AAT CAC

GAT AAT GAT GAC AGT GAT GCA AGC GAT GCA GTT GAT GAA GAT ATT GAG

ATA CTT GAG TCT TAT AGT GAT TTG AAT AAA TTT AAT GAG ATG TTA ACA

GAA CAA TTA AAT AAA AAT AAG GAT GGA TAT GTT GTT TTC GTT ACT GAA

TTA TTT GGT GAA CAT CTT TTT CAA TAT ATT AAT AAA ACG AAT GAA AAT

GAA GAT ACG CGT GTA AGA GAT GAA GAT AAA AAA ATA ATT ATG TTT GAA

TTT TTA AAG TTA TTA ATA AAA TTA CAT AAT GCA GGA TTC CTA CAT CTT

GAT ATA TCT CCT GAA AAT ATA TTG ATA GAA AAT AAT GGT GAC TTA CGC

TTA TGT GAT GTA GCT AAA TGT GCT CCA ATG TAT ACA CAT AAT TTA AGA

CAT ATT AAA GGA AAT GGA AAC CAT TTG TAT TCA TTT CAA TCT TGT CAA

CCT TGT GTT GGA AAA ATC CCA TGT ATT CCA AAA GAC TGT TGG GAT ATT

ATT AGA GAA CAT ATA AAA TTA AAA ATA GAT AAT CCT TTT GAA CAT TTA

TCA ACT ATT ACT GAT CAA GAA GAA AGA AAA AAA TAT TAT TTT CAT GTA

CAT TGT GTA GAT AAA TAT ATG CTT CGT ATA TTT TAT ATA TGG ATA TGG


AAT TTA AAT TAT ATA TGG AAA CCA GCT GAC CCA CCA AAT GAT AGA ACA

TTT AAT AAT TTC TTA AAA TAT AAT TTA AAT ATA AAT GTA TTT CAG TTA

GCC CAA CAA TGG CCA AAA GGT CTC AAA GAT ATA ATT AAC GTA AGA TAT

ATA AGA AAA AAA AAA AAA AAA AAA AAA ATA ATA ATA ATA ATA ATG ATG

TTT TAT ATA GTA TGT GCA TAG TGAATGTTTT TTTTTTATAT TTTAATACAT


```
GAAATGATAT ATATATATAT ATATATATAT ATATTTTCTC TTTATAGAAA
TTATTAAGCC TAGAAAGTAG AATGAAGACA GACTTAAATG AATTAACTGA
ACATCCATGG TGGATTAATC AAGATTAATT TTAATTTTTT AAGTATTATA
TGAATATTTA TTATTAGTAG ACTTTATATA TAACAATAAA TATTGACACG
TGCAATATTA AAAAAAAAAA AAAAAAAAA AAAAAAAGA ATTAAAAAGG
AATGTTTTAT TGTTTAGGAC TATATGGAAA AATAATAATA TATATATGTT
TCCACATTAA AAA
```

the boxed section corresponding to the nucleotide sequence constituting the "n" repetitions of 18 bp, "n" being sufficient for the sequence formed to code for an antigen liable to be recognized by antibodies raised against *P. falciparum* or of inducing such antibodies,

the notation (...) representing nucleotide triplets or repeated codons.

**8.** Nucleotide sequence of P. *falsiparum,* characterized in that it corresponds to either of the following sequences I and II or to a part of the sequences coding for an antigen recognized by antibodies raised against *P. falciparum* or capable of inducing or of contributing towards the *in vivo* production of such antibodies.

I :

CT

GAG GAA TGT CAA GCG GAA GTT TAT TTA TTT GTT AAA AAG ATA CCT ATA

GAG GTA TGG ATA ACA CAG TAC AAC TTG ATG AAT GAT AAT GAT GGA GAA

TAT TTA TTA GAT CGG GAA AAT TTT ATT ATC GAA GCA GTA GCC TGC GCT

TAT TTA AGC GAG CAT TAT CCT GGA CTC ACA CCT AAA TTG TAC AAG GTA

TTA TAT GAG CCT GAA TGT GCT AAC TGT AAT GAA CAG GAT AAG AAT ATG

AGT GAA GAT AAT

II:                    AAA AAT AAT AAC AAT AAT AAG GAT AAT AAA AAT GAC

GAT AAT GAT GAC ACT GAT GCA AGC GAT GCA GTT GAT GAA GAT ATT GAG

ATA CTT GAG TCT TAT ACT GAT TTC AAT AAA TTT AAT GAG ATG TTA ACA

GAA CAA TTA AAT AAA AAT AAG GAT GGA TAT GTT GTT TTC GTT ACT GAA

TTA TTT GGT GAA GAT CTT TTT CAA TAT ATT AAT AAA AGC AAT GAA AAT

GAA GAT ACG CCT GTA AGA GAT GAA GAT AAA AAA ATA ATT ATG TTT GAA

TTT TTA AAG TTA TTA ATA AAA TTA CAT AAT GCA GGA TTG GTA CAT CTT

GAT ATA TCT CCT GAA AAT ATA TTG ATA GAA AAT AAT GGT GAC TTA CCC

TTA TGT GAT CTA GCT AAA TGT GCT CCA ATG TAT ACA CAC AAT TTA AGA

CAT ATT AAA GGA AAT GGA AAC GAT TTG TAT TCA TTT CAA TCT TGT CAA

CCT TGT GTT GGA AAA ATC CCA TCT ATT CCA AAA GAG TGT TGG GAT ATT

ATT ACA GAA CAT ATA AAA TTA AAA ATA GAT AAT CCT TTT GAA CAT TTA

TCA ACT ATT ACT GAT CAA GAA GAA ACA AAA AAA TAT TAT TTT GAT GTA

CAT TGT GTA GAT AAA TAT ATG CTT GGT ATA TTT TAT ATA TCG ATA TGG

AAT TTA AAT TAT ATA TGG AAA CGA GCT GAC CCA CCA AAT GAT AGA ACA

TTT AAT AAT TTC TTA AAA TAT AAT TTA AAT ATA AAT GTA TTT CAG TTA

GCC CAA CAA TGG CCA AAA GGT CTC AAA GAT ATA ATT AAC GTA AGA TAT

ATA AGA AAA AAA AAA AAA AAA AAA AAA ATA ATA ATA ATA ATA ATC ATG

TTT TAT ATA GTA TGT GGA TAC TGAATGTTTT TTTTTTATAT TTTAATACAT

GAAATGATAT ATATATATAT ATATATATAT ATATTTTCTC TTTATAGAAA
TTATTAAGCC TAGAAAGTAG AATGAAGACA GACTTAAATC AATTAACTGA
ACATCCATGG TGGATTAATC AAGATTAATT TTAATTTTTT AAGTATTATA
TGAATATTTA TTATTAGTAG ACTTTATATA TAACAATAAA TATTGACACG
TGCAATATTA AAAAAAAAA AAAAAAAAA AAAAAAAGA ATTAAAAAGC
AATGTTTTAT TGTTTAGGAC TATATGGAAA AATAATAATA TATATATGTT
TCCACATTAA AAA

9. Antigen of *P. falciparum* capable of inducing the formation of antibodies, in particular of antibodies which protect against *P. falciparum,* characterized in that it is the product of translation of a sequence according to any one of Claims 1 to 8.

10. Antigen of *P. falciparum,* characterized in that:

- it is capable of inducing antibodies and in particular antibodies which protect against *P. falciparum,*
- it comprises an amino acid sequence containing an oligopeptide consisting of n repetitions of identical or non-identical peptides, corresponding to the formula Asp b His cys Ser, in which b is any amino acid and preferably Asn, His, Ser or Cys or a variant obtained by substitution of one or more amino acids, provided that the antigenic and/or immunogenic properties of the antigen formed are conserved,
- n is sufficient for the oligopeptide formed to be capable of inducing, *in vivo,* antibodies which recognize the antigen coded by the nucleotide sequence of *P. falciparum* corresponding to the following restriction map :

11. Antigen of *P. falciparum* according to Claim 10, characterized in that it comprises the following amino acid sequence:

EP 0 542 845 B1

Glu Glu Cys Gln Gly Glu Val Tyr Leu Phe Val Lys Lys Ile Pro Ile

Glu Val Trp Ile Arg Gln Tyr Asn Leu Met Asn Asp Asn Asp Gly Glu.

Tyr Leu Leu Asp Gly Glu Asn Phe Ile Met Glu Ala Val Ala Cys Ala

Tyr Leu Ser Glu His Tyr Pro Gly Leu Thr Pro Lys Leu Tyr Lys Val

Leu Tyr Glu Pro Glu Cys Ala Asn Cys Asn Glu Glu Asp Lys Asn Met

Ser Glu Asp Asn His Lys Lys Asp Ser Lys His Lys Gly Asp Ser Asn

His Lys Ser Asp Ser Asn His Lys Ser Asp Ser Asn His Lys Ser Asp

Ser Asn His Lys Ser Asp Ser Asn His Lys Ser Gly Ser Asn His Lys

Ser Asp Cys Asn His Lys Ser Gly Ser Asn His Lys Ser Gly Ser Asn

His Lys Ser Asp Ser Asn His Gln Ser Asp Cys Asn ... ... ... ...

... ... ... ... ... ... ... ... ... Asp His Asn His Lys Ser Asp

His Asn His Lys Ser Asp Asn Asn His Lys Ser Asp His Asn His Lys

Ser Asp His Lys Lys Asn Asn Asn Asn Asn Lys Asp Asn Lys Asn Asp

Asp Asn Asp Asp Ser Asp Ala Ser Asp Ala Val Asp Glu Asp Ile Glu

Ile Leu Glu Ser Tyr Ser Asp Leu Asn Lys Phe Asn Glu Met Leu Thr

Glu Gln Leu Asn Lys Asn Lys Asp Gly Tyr Val Val Leu Val Thr Glu

Leu Phe Gly Glu Asp Leu Phe Gln Tyr Ile Asn Lys Arg Asn Glu Asn

Glu Asp Thr Arg Val Arg Asp Glu Asp Lys Lys Ile Ile Met Phe Glu

Phe Leu Lys Leu Leu Ile Lys Leu His Asn Ala Gly Leu Val His Leu

Asp Ile Ser Pro Glu Asn Ile Leu Ile Glu Asn Asn Gly Glu Leu Arg

Leu Cys Asp Leu Ala Lys Cys Ala Pro Met Tyr Thr His Asn Leu Arg

His Ile Lys Gly Asn Gly Asn Asp Leu Tyr Ser Phe Gln Ser Cys Gln

Pro Cys Val Gly Lys Ile Pro Cys Ile Pro Lys Glu Cys Trp Asp Ile

Ile Arg Glu His Ile Lys Leu Lys Ile Asp Asn Pro Phe Glu His Leu

Ser Thr Ile Thr Asp Gln Glu Glu Arg Lys Lys Tyr Tyr Phe Asp Val

His Cys Val Asp Lys Tyr Met Leu Gly Ile Phe Tyr Ile Trp Ile Trp

Asn Leu Asn Tyr Ile Trp Lys Arg Ala Asp Pro Pro Asn Asp Arg Thr

Phe Asn Asn Phe Leu Lys Tyr Asn Leu Asn Ile Asn Val Phe Gln Leu

Ala Gln Gln Trp Pro Lys Gly Leu Lys Asp Ile Ile Asn Val Arg Tyr

Ile Arg Lys Lys Lys Lys Lys Lys Lys Ile Ile Ile Ile Ile Met Met

Phe Tyr Ile Val Cys Ala,

the boxed section corresponding to the peptide sequence constituting the "n" repetitions of 6 amino acids, "n" being defined as in Claim 10,

the notation (...) representing an amino acid of the repeat sequence.

12. Antigen of *P. falciparum* according to Claim 10, characterized in that it comprises one of the following amino acid sequences R23, R45 or R51:

R23

LysSerAspSerAsnHisLysSerAspHisAsnHisLysSerAspSerAsn

HisMetSerAspHisAsnHisMetSerAspHisAsnHisLysSerAspHisAsnHisLys

SerAspAsnAsnHisLysSerAspSerAsnHisLysSerAspSerAsnHisLysSerAsp

SerAsnHisLysSerAspHisAsn

R51

HisAsnHisMetSerAspHisAsnHisLysSerAspAsnAsnHisLysSerAspHis

AsnHisLysSerAspSerAsnHisMetSerAspHisAsnHisMetSerAspHisAsnHis

LysSerAspHisAsnHisLysSerAspHisAsnHisLysSerAspAsnAsnHisLysSer

AspSerAsnHisLysSer

R45

HisLysSerAspAsnAsnHisLysSerAspSerAsnHisLysSerAspSerAsn

HisLysSerAspSerAsnHisLysSerAspHisAsnHisLysSerAspHisLysHisMet

SerAspAsnAsnHisLysSerAspAsnAsnHisLysSerAspAsnAsnHisLysSerAsp

HisAsnHisLysSerAspAsnAsnHisLysSerAspHisAsnHisLysSerAspSerAsn

HisLysSerAspSerAsnHisLysSerAspSerAsnHisLysSerAspSerAsnHisLys

SerAspAsnAsnHisLysSerAspHisAsnHis

13. Antigen of *P. falciparum* according to any one of Claims 9 to 12, characterized in that it is capable of inducing, *in vivo,* the production of protective cytophilic antibodies, in particular antibodies IgG$_1$ and IgG$_3$.

14. Recombinant vector for cloning and/or expression, characterized in that it comprises a nucleotide sequence according to any one of Claims 1 to 8, where appropriate under the control of a promoter and regulation elements for expression in a specific cell host.

15. Recombinant vector according to Claim 14, characterized in that the vector used is pMS, λgt11 or pGEX.

16. Recombinant polypeptide, characterized in that it comprises an antigenic sequence according to any one of Claims 9 to 13, in combination with a polypeptide, in particular a polypeptide characteristic of a blood stage or liver stage of *P. falciparum* infection, for example the antigen 96TR or alternatively a polypeptide or a protein carrying in particular β-galactosidase, tetanus anatoxin, ovalbumin, serum albumin, glutathione transferase or alternatively a synthetic carrier molecule.

17. Recombinant cell host, characterized in that it is transformed by a vector according to either of Claims 14 and 15, under conditions allowing the expression of the sequence of *P. falciparum* according to any one of Claims 1 to 8, and in that it is in particular a bacterium such as *E. coli,* a yeast, an insect cell or a mammalian cell such as CHO.

18. Cell host according to Claim 17, characterized in that it is the strain SB275 filed at the CNCM under No. I-986 of 27 July 1990.

19. Cell host according to Claim 18, characterized in that it is the strain SB363 filed at the CNCM under No. I-987 on 27 July 1990.

20. Vaccine composition, characterized in that it comprises, as main active agent, an antigen according to any one of Claims 9 to 14.

21. vaccine composition according to claim 20, *characterized* in that it also contains another antigen from the blood stage or liver stage of *P. falciparum.*

22. Antigen, characterized in that it is recognized by antibodies formed against the sequence according to Claim 11, in that it has a molecular weight of about 160 kDa and in that it is present at an early stage of infection by *P. falciparum*.

23. Peptide sequence, characterized in that it is recognized by antibodies directed against *P. falciparum* and where appropriate, in that it is capable of inducing the formation of antibodies against *P. falciparum,* or of contributing towards the formation of such antibodies, and in particular in that it is the following sequence HKSDNNHKSDSNHKSDSN.

24. DNA or RNA fragments which may be used as primers, consisting of about 8 to 25 nucleotides which are identical to a sequence of the same length from a sequence according to any one of Claims 1 to 8, or DNA or RNA fragments capable of hybridizing with a sequence according to any one of Claims 1 to 8 in a medium corresponding to the following conditions: 6 x SSC, 2.5% milk, 100 µg/ml herring DNA, 65°C with washing steps in a medium containing 6 x SSC and 0.5 SSC.

GENE R45

≈80 répètitions
18 bp

Dde I

A   Ac   A        Ac        Ac              Ac        Dde I

R R                          R    R        Rp          R    R

A2

ORF

___ 250bp

A  :  Alu I
Ac :  Acc I
R  :  Rsal
·  :  codon stop
Rp :  ≈ 7 répétitions 45 bp

**FIGURE 1**

EP 0 542 845 B1

DdeI
CT

GAG GAA TGT CAA GGG GAA GTT TAT TTA TTT GTT AAA AAG ATA CCT ATA

GAG GTA TGG ATA AGA CAG TAC AAC TTG ATG AAT GAT AAT GAT GGA GAA

TAT TTA TTA GAT GGG GAA AAT TTT ATT ATG GAA GCA GTA GCC TGC GCT

TAT TTA AGC GAG CAT TAT CCT GGA CTC ACA CCT AAA TTG TAC AAG GTA

TTA TAT GAG CCT GAA TGT GCT AAC TGT AAT GAA GAG GAT AAG AAT ATG

AGT GAA GAT AAT CAT AAG AAG GAT AGT AAA CAT AAG GGG GAT AGT AAT

CAC AAA AGT GAT AGT AAT CAC AAA AGT GAT AGT AAT CAC AAA AGT GAT

AGT AAT CAT AAA AGT GAT AGT AAT CAT AAA AGT GGT AGT AAT CAC AAA

AGT GAT TGT AAT CAC AAG AGT GGT AGT AAT CAC AAG AGT GGT AGT AAT

CAC AAG AGT GAT AGT AAT CAT CAA AGT GAT TGT AAT ... ... ... ...


≈ 80 REPETITIONS 18 bp


... ... ... ... ... ... ... ... ... GAT CAT AAT CAC AAA AGC GAT

CAT AAC CAC AAG AGC GAT AAT AAC CAC AAG AGT GAT CAT AAT CAC AAA

AGT GAT CAT AAA AAA AAT AAT AAC AAT AAT AAG GAT AAT AAA AAT GAC

GAT AAT GAT GAC AGT GAT GCA AGC GAT GCA GTT GAT GAA GAT ATT GAG

ATA CTT GAG TCT TAT AGT GAT TTG AAT AAA TTT AAT GAG ATG TTA ACA

GAA CAA TTA AAT AAA AAT AAG GAT GGA TAT GTT GTT TTG GTT ACT GAA

TTA TTT GGT GAA GAT CTT TTT CAA TAT ATT AAT AAA AGG AAT GAA AAT

GAA GAT ACG CGT GTA AGA GAT GAA GAT AAA AAA ATA ATT ATG TTT GAA

TTT TTA AAG TTA TTA ATA AAA TTA CAT AAT GCA GGA TTG GTA CAT CTT

GAT ATA TCT CCT GAA AAT ATA TTG ATA GAA AAT AAT GGT GAG TTA CGC

TTA TGT GAT CTA GCT AAA TGT GCT CCA ATG TAT ACA CAC AAT TTA AGA


**FIGURE 2**

CAT ATT AAA GGA AAT GGA AAC GAT TTG TAT TCA TTT CAA TCT TGT CAA

CCT TGT GTT GGA AAA ATC CCA TGT ATT CCA AAA GAG TGT TGG GAT ATT

ATT AGA GAA CAT ATA AAA TTA AAA ATA GAT AAT CCT TTT GAA CAT TTA

TCA ACT ATT ACT GAT CAA GAA GAA AGA AAA AAA TAT TAT TTT GAT GTA

CAT TGT GTA GAT AAA TAT ATG CTT GGT ATA TTT TAT ATA TGG ATA TGG


AAT TTA AAT TAT ATA TGG AAA CGA GCT GAC CCA CCA AAT GAT AGA ACA

TTT AAT AAT TTC TTA AAA TAT AAT TTA AAT ATA AAT GTA TTT CAG TTA

GCC CAA CAA TGG CCA AAA GGT CTC AAA GAT ATA ATT AAC GTA AGA TAT

ATA AGA AAA AAA AAA AAA AAA AAA AAA ATA ATA ATA ATA ATA ATG ATG

TTT TAT ATA GTA TGT GCA TAG TGAATGTTTT TTTTTTATAT TTTAATACAT
              * * *

GAAATGATAT ATATATATAT ATATATATAT ATATTTTCTC TTTATAGAAA
TTATTAAGCC TAGAAAGTAG AATGAAGACA GACTTAAATG AATTAACTGA
ACATCCATGG TGGATTAATG AAGATTAATT TTAATTTTTT AAGTATTATA
TGAATATTTA TTATTAGTAG ACTTTATATA TAACAATAAA TATTGACACG
TGCAATATTA AAAAAAAAAA AAAAAAAAAA AAAAAAAGA ATTAAAAAGG
AATGTTTTAT TGTTTAGGAC TATATGGAAA AATAATAATA TATATATGTT
TCCACATTAA AAA

POLY A (23-25)

● CODONS STOP (3 PHASES)


**FIGURE 2 (suite)**

Glu Glu **Cys** Gln Gly Glu Val Tyr Leu Phe Val Lys Lys Ile Pro Ile

Glu Val **Trp** Ile Arg Gln Tyr Asn Leu Met Asn Asp Asn Asp Gly Glu

Tyr Leu **Leu** Asp Gly Glu Asn Phe Ile Met Glu Ala Val Ala Cys Ala

Tyr Leu Ser Glu His Tyr Pro Gly Leu Thr Pro Lys Leu Tyr Lys Val

Leu Tyr **Glu** Pro Glu Cys Ala Asn Cys Asn Glu Glu Asp Lys Asn Met

Ser Glu Asp Asn His Lys Lys Asp Ser Lys His Lys Gly Asp Ser Asn

His Lys Ser Asp Ser Asn His Lys Ser Asp Ser Asn His Lys Ser Asp

Ser Asn His Lys Ser Asp Ser Asn His Lys Ser Gly Ser Asn His Lys

Ser Asp Cys Asn His Lys Ser Gly Ser Asn His Lys Ser Gly Ser Asn

His Lys Ser Asp Ser Asn His Gln Ser Asp Cys Asn ... ... ... ...

## ∼ 80 REPETITIONS 6 aa

... ... ... ... ... ... ... ... ... Asp His Asn His Lys Ser Asp

His Asn His Lys Ser Asp Asn Asn His Lys Ser Asp His Asn His Lys

Ser Asp His Lys Lys Asn Asn Asn Asn Asn Lys Asp Asn Lys Asn Asp

Asp Asn Asp Asp Ser Asp Ala Ser Asp Ala Val Asp Glu Asp Ile Glu

Ile Leu Glu Ser Tyr Ser Asp Leu Asn Lys Phe Asn Glu Met Leu Thr

Glu Gln Leu Asn Lys Asn Lys Asp Gly Tyr Val Val Leu Val Thr Glu

Leu Phe Gly Glu Asp Leu Phe Gln Tyr Ile Asn Lys Arg Asn Glu Asn

Glu Asp Thr Arg Val Arg Asp Glu Asp Lys Lys Ile Ile Met Phe Glu

Phe Leu Lys Leu Leu Ile Lys Leu His Asn Ala Gly Leu Val His Leu

Asp Ile Ser Pro Glu Asn Ile Leu Ile Glu Asn Asn Gly Glu Leu Arg

Leu Cys Asp Leu Ala Lys Cys Ala Pro Met Tyr Thr His Asn Leu Arg

His Ile Lys Gly Asn Gly Asn Asp Leu Tyr Ser Phe Gln Ser Cys Gln

Pro Cys Val Gly Lys Ile Pro Cys Ile Pro Lys Glu Cys Trp Asp Ile

Ile Arg Glu His Ile Lys Leu Lys Ile Asp Asn Pro Phe Glu His Leu

Ser Thr Ile Thr Asp Gln Glu Glu Arg Lys Lys Tyr Tyr Phe Asp Val

His Cys Val Asp Lys Tyr Met Leu Gly Ile Phe Tyr Ile Trp Ile Trp

Asn Leu Asn Tyr Ile Trp Lys Arg Ala Asp Pro Pro Asn Asp Arg Thr

**FIG 3**

```
Phe Asn Asn Phe Leu Lys Tyr Asn Leu Asn Ile Asn Val Phe Gln Leu
Ala Gln Gln Trp Pro Lys Gly Leu Lys Asp Ile Ile Asn Val Arg Tyr
Ile Arg Lys Lys Lys Lys Lys Lys Ile Ile Ile Ile Ile Met Met
Phe Tyr Ile Val Cys Ala ***
```

## FIGURE 3 (suite et fin)

AC

```
AAA AGT GAT AGT AAC CAC AAG AGC GAT CAT AAT CAC AAG AGT GAT AGT
Lys Ser Asp Ser Asn His Lys Ser Asp His Asn His Lys Ser Asp Ser

AAC CAC ATG AGC GAT CAT AAC CAC ATG AGC GAT CAT AAC CAC AAG AGC
Asn His Met Ser Asp His Asn His Met Ser Asp His Asn His Lys Ser

GAT CAT AAC CAC AAG AGC GAT AAT AAC CAC AAA AGT GAT AGT AAC CAC
Asp His Asn His Lys Ser Asp Asn Asn His Lys Ser Asp Ser Asn His

AAA AGT GAT AGT AAC CAC AAA AGT GAT AGT AAC CAC AAG AGC GAT CAT
Lys Ser Asp Ser Asn His Lys Ser Asp Ser Asn His Lys Ser Asp His

AAT C
Asn
```

**FIGURE 4**

```
CAC AAG AGC GAT AAT AAC CAC AAA AGT GAT AGT AAC CAC AAA AGT GAT
His Lys Ser Asp Asn Asn His Lys Ser Asp Ser Asn His Lys Ser Asp
1              15              30              45

AGT AAC CAC AAA AGT GAT AGT AAC CAC AAG AGC GAT CAT AAT CAC AAG
Ser Asn His Lys Ser Asp Ser Asn His Lys Ser Asp His Asn His Lys
            60              75              90

AGC GAT CAT AAG CAC ATG AGC GAT AAT AAC CAC AAG AGC GAT AAT AAT
Ser Asp His Lys His Met Ser Asp Asn Asn His Lys Ser Asp Asn Asn
        105             120             135

CAC AAG AGC GAT AAT AAT CAC AAG AGC GAT CAT AAT CAC AAG AGC GAT
His Lys Ser Asp Asn Asn His Lys Ser Asp His Asn His Lys Ser Asp
    150             165             180

AAT AAT CAC AAG AGC GAT CAC AAT CAC AAG AGT GAT AGT AAT CAC AAG
Asn Asn His Lys Ser Asp His Asn His Lys Ser Asp Ser Asn His Lys
195             210             225             240

AGT GAT AGT AAC CAC AAG AGT GAT AGT AAC CAC AAG AGT GAT AGT AAC
Ser Asp Ser Asn His Lys Ser Asp Ser Asn His Lys Ser Asp Ser Asn
            255             270             285

CAC AAG AGT GAT AAT AAC CAT AAG AGC GAT CAT AAT CAC
His Lys Ser Asp Asn Asn His Lys Ser Asp His Asn His
        300             315
```

## FIGURE 5

```
CAC AAT CAC ATG AGC GAT CAT AAT CAC AAG AGT GAT AAT AAC CAT AAG
His Asn His Met Ser Asp His Asn His Lys Ser Asp Asn Asn His Lys
1               15                  30                  45

AGT GAT CAT AAT CAC AAG AGT GAT AGT AAC CAC ATG AGC GAT CAT AAC
Ser Asp His Asn His Lys Ser Asp Ser Asn His Met Ser Asp His Asn
            60                  75                  90

CAC ATG AGC GAT CAT AAC CAC AAG AGC GAT CAT AAT CAC AAG AGC GAT
His Met Ser Asp His Asn His Lys Ser Asp His Asn His Lys Ser Asp
        105                 120                 135

CAT AAC CAC AAG AGC GAT AAT AAC CAC AAA AGT GAT AGT AAC CAC AAA
His Asn His Lys Ser Asp Asn Asn His Lys Ser Asp Ser Asn His Lys
    150                 165                 180

AGT GA
Ser
195
```

**FIGURE 6**

64

FIGURE 7

EP 0 542 845 B1

FIGURE 8

66

FIGURE 9

EP 0 542 845 B1